# EUROPEAN PATENT SPECIFICATION

(11) **EP 4 069 866 B1**
(45) Date of publication and mention of the grant of the patent: **22.05.2024**
(21) Application number: 20821430.4
(22) Date of filing: 02.12.2020
(51) Int. Cl.: C12Q 1/6809, C12Q 1/689

(54) **METHODS FOR IDENTIFYING MICROBES IN A CLINICAL AND NON-CLINICAL SETTING**
VERFAHREN ZUR IDENTIFIZIERUNG VON MIKROBEN IN EINER KLINISCHEN UND NICHTKLINISCHEN UMGEBUNG
PROCÉDÉS D'IDENTIFICATION DE MICROBES DANS UN ENVIRONNEMENT CLINIQUE ET NON CLINIQUE

(30) Priority: 02.12.2019 EP 19212955
(43) Date of publication of application: 12.10.2022
(73) Proprietor: Biomiris Capital Group B.V., 1098 XG Amsterdam (NL)
(72) Inventor: BUDDING, Andries Edward, 1098 XG Amsterdam (NL)
(74) Representative: V.O.
(86) International application number: PCT/NL2020/050753
(87) International publication number: WO 2021/112673

(56) References cited:
- US-A1- 2017 321 257
- ARIANE T. PIETZKA ET AL: "Gene Scanning of an Internalin B Gene Fragment Using High-Resolution Melting Curve Analysis as a Tool for Rapid Typing of Listeria monocytogenes", THE JOURNAL OF MOLECULAR DIAGNOSTICS, vol. 13, no. 1, 1 January 2011 (2011-01-01), pages 57-63, XP055692291, ISSN: 1525-1578, DOI: 10.1016/j.jmoldx.2010.11.002
- PANGASA A ET AL: "High resolution melting-curve (HRM) analysis for the diagnosis of cryptosporidiosis in humans", MOLECULAR AND CELLULAR PROBES, ACADEMIC PRESS, LONDON, GB, vol. 23, no. 1, 1 February 2009 (2009-02-01), pages 10-15, XP025910704, ISSN: 0890-8508, DOI: 10.1016/J.MCP.2008.10.003 [retrieved on 2008-10-29]
- Stephanie I. Fraley ET AL: "Nested Machine Learning Facilitates Increased Sequence Content for Large-Scale Automated High Resolution Melt Genotyping", Scientific Reports, vol. 6, 18 January 2016 (2016-01-18), page 19218, XP055305851, DOI: 10.1038/srep19218
- Panousis Konstantinos ET AL: "Poor predictive value of broad-range PCR for the detection of arthroplasty infection in 92 cases", ACTA ORTHOPAEDICA, vol. 76, no. 3, 1 January 2005 (2005-01-01), pages 341-346, XP093105042, GB ISSN: 1745-3674, DOI: 10.1080/00016470510030805
- BHASKARAN ARCHANA ET AL: "Interpretation of positive molecular tests of common viruses in the cerebrospinal fluid", DIAGNOSTIC MICROBIOLOGY AND INFECTIOUS DISEASE, ELSEVIER, AMSTERDAM, NL, vol. 77, no. 3, 12 September 2013 (2013-09-12), pages 236-240, XP028736254, ISSN: 0732-8893, DOI: 10.1016/J.DIAGMICROBIO.2013.07.017
- Gascoyne-Binzi D M ET AL: "False negative polymerase chain reaction on cerebrospinal fluid samples in tuberculous meningitis", JOURNAL OF NEUROLOGY NEUROSURGERY & PSYCHIATRY., vol. 67, no. 2, 1 August 1999 (1999-08-01) , pages 249-260, XP093043397, GB ISSN: 0022-3050, DOI: 10.1136/jnnp.67.2.250

## Description

### FIELD OF THE INVENTION

The invention is in the field of microbial diagnostics, in particular to rapid identification of microbes in samples at the species or strain level, in particular bacteria in clinical and non-clinical samples. The invention relates to methods of rapid microbial identification based on DNA analysis and systems for performing such methods.

### BACKGROUND OF THE INVENTION

Increasing population, urbanization and global connectivity has hugely increased the efficacy of dissemination of agents of infectious disease. This is clearly exemplified by the very recent and very rapid worldwide spread of high-risk clones of multidrug-resistant bacteria, which have now become an important public health threat. Therefore, early detection protocols for hazardous bacteria are ever more needed. In addition, and in line with the 'One Health' approach of the World Health Organization, laboratory diagnosis of infections in livestock or microbial contaminations in non-clinical settings, such as microbiological testing of sterile products in the food sector or pharmaceutical industry, should include improved protocols for accurate detection and identification of microbial pathogens or contaminants, while reducing turnaround times and costs.

As classic culture techniques are insufficiently sensitive and time consuming (>24 hours), focus in recent years has shifted to culture-independent methods, including DNA-based molecular methods in order to detect, characterize, and study the epidemiology and clinical impact of such microbes. A large number of DNA-based diagnostic approaches are currently available. Although this progress is encouraging, any single diagnostic platform cannot fully address the need for actionable data with short turnaround times in all settings.

Although the detection of microbes has been revolutionized by the development and application of species-specific and strain-specific polymerase chain reaction (PCR) procedures for high-specificity amplification and detection of even the smallest amounts of microbial DNA from samples, such methods require insight in which microbe can be expected in a clinical sample. A negative test result provides limited information, as only the absence of one or a few specific species has been shown. Furthermore, in situations where the causal infectious agent is hard to predict, evades detection by mutation, or is in fact not a single species or type of microbe, specific DNA-based diagnostic approaches have limited applicability. It is clear that high specificity in PCR amplification has its pros and cons when it comes to clinical and non clinical diagnostics.

And despite their high specificity, verification of amplicon identity is still unavoidable in such methods, to rule out amplification of non-target sequences. Amplicon verification may include the use of hybridization probes, the detection of restriction sites in the amplicon, confirmation of the expected length of the amplicon in number of nucleotides, or the sequencing of the amplicon.

Currently, methods of bacterial identification by next-generation DNA sequencing are unsuitable for clinical application because these sequencing approaches require at least about 24 hours before results are available, whereas treatment decisions need to be made within the shortest possible time frames. Moreover, common sequencing approaches allow only for relatively short DNA fragments (e.g. about 300 bps) to be analysed, which is too short to allow for a sufficient resolution on the species or strain level, whereas sequencing techniques that allow for the sequencing of longer fragments are either too expensive, require large sample batches to become affordable or are too inaccurate to be implemented in clinical routine.

US2017321257A1 describes methods and oligonucleotide reagents for identification of bacteria by high resolution melting (HRM) analysis. Pietzka et al. 2011 (J Mol Diagn. 13(1):57-63) disclose a method of typing clinical *L. monocytogenes* isolates by high-resolution melting (HRM) curve analysis of a specific locus of the internalin B gene (inlB). Pangasa et al. 2009 (Mol. Cell. Probes 23(1):10-15) disclose a polymerase chain reaction (PCR)-coupled high resolutionmelting-curve (HRM) analysis method, utilizing the second internal transcribed spacer (ITS-2) of nuclearribosomal DNA as the genetic marker, for the diagnosis of *Cryptosporidium hominis*, *C. parvum* or *C.meleagridis* infection.

To summarize the situation in clinical diagnostics, there are five key issues which dictate the applicability of any assay: (1) Scope, (2) Speed, (3) Sensitivity, (4) Specificity, and (5) Scalability. Classic culture-based microbial diagnostics score well on three of five items: scope (many different micro-organisms can be detected), sensitivity (up to a single bacterial cell can be detected) and specificity (determination of bacterial species/strain is accurate). However, culture does not score well for speed and scalability (high throughput).

Current qPCR-based diagnostics score well on four of five items: speed, sensitivity specificity and scalability. However these assays are extremely limited in scope: a single qPCR can detect only a single or a few target micro-organisms, which means an endless array of qPCRs would be needed to achieve a scope somewhat comparable to that of culture.

Moreover, even in such a setting a negative result (no microorganism of any species present) could not be given. Negative results are of extreme importance in clinical microbiology, as some of the most important clinical decisions are made based on negative results, examples of which include finding an alternative cause for a disease than an infection, stopping antibiotics, discharging patients, removal of indwelling devices (postoperative drains, and iv catheters), and decisions on maintaining highly important foreign bodies in a patient (prosthetic joints, osteosynthesis plates and others). Because of the importance of negative results and the inability of qPCR to accurately provide these, culture is still maintained as the mainstay of microbiological diagnostics, despite its obvious limitations.

It is clear then, that it would be highly desirous to provide for a cost-effective and efficient PCR-based microbial detection system that scores well on all five key issues, in particular for clinical applicability.

### SUMMARY OF THE INVENTION

The present inventor has now unexpectedly discovered a method for broad microbial identification that can be also applied in the clinic. The present inventor has realized that the development of still more species- and strain-specific PCR amplification tests, to add to the existing array of available diagnostic tests for specific pathogens, is not going to solve the problem. The present inventor has adopted the approach of combining less-specific amplification protocols as a first part, with highly specific detection/verification procedures as a second part, wherein both parts can be run at high throughput.

The method of the present invention uses a more "universal" amplification of a microbial rRNA internal transcribed spacer (ITS) region from genomic DNA, instead of amplifying a species- or strain-specific sequence. The identity of the microbe from which this ITS region is amplified, is then determined by combining information of amplicon length with the DNA melting curve of the amplicon. It was surprisingly found that this method provided for a very high level of resolution at the species and strain level, could be universally applied to a large number of microbial organisms, and resulted in a build-up of a database with data on amplicon length and melting curve annotated to species and strains that improved accuracy of identification of microbes in subsequent samples. Most surprisingly, this relatively simple method was capable of providing clinically relevant information based upon which the clinician could make a treatment decision, even for the highly important negative samples (no microorganism detected).

In a method of the invention, the amplification of an rRNA internal transcribed spacer (ITS) region of microbial genomic DNA is essentially not species- or strain-specific. Although highest resolution at the strain level may be attained by the use of species-specific amplification protocols, in a method of the invention, it is intended that the amplification procedure targets an rRNA ITS region of a large number of microbial species (covering a broader range of several taxonomic genera, families, orders, classes, phyla, or even kingdoms), such that the amplicon is derived from a potentially large group of microbes. The "universal" DNA amplification is therefore preferably performed by using higher taxon level or group-specific primers such as genus-specific, family-specific, order-specific, class-specific, phylum-specific, kingdom-specific and/or domain-specific amplification primers for amplifying an rRNA ITS region of microbial genomic DNA suspected of being present in a sample. By choosing a universal amplification approach, all bacterial species can be detected, negating the need of many separate qPCRs to achieve a broad scope. Moreover, because all bacterial species should be amplified, negative results mean there are no bacteria present in a sample and they can indeed be used as a sound base for clinical decisions. Hitherto, such a universal PCR approach has not been applied in the clinic for microbial identification because such broad PCR amplification could result in the amplification of multiple PCR amplicons derived from multiple species, each having a different length and a different nucleotide sequence, which does not result in actual identification in all situations as specificity levels are considered too low for clinical purpose. Moreover, the nature of clinical specimens is often that human DNA is present in much higher abundance than bacterial DNA. As these the differences in concentration can be extraordinarily high (it is not uncommon that human DNA outnumbers potential bacterial DNA by a factor of 10¹⁰), even with very specific bacterial amplification primers, cross reactivity is common, leading to non-specific amplification and false positive results.

Unexpectedly, the present inventor has found a way to make a universal PCR approach available for clinical use despite above mentioned problems by combining in said universal PCR approach two techniques that together provide for a clinically sufficient sensitivity and specificity, namely the technique of (i) high resolution melting curve analysis (hrMCA) of amplified PCR products, and (ii) PCR amplicon length analysis by performing, for instance, capillary electrophoretic separation and subsequent fragment length analysis of amplified PCR products (i.e. amplicons).

The present invention now provides a method for detecting and identifying a microorganism at species or strain level in a biological sample by polymerase chain reaction (PCR), said method comprising the steps of:
a) providing a biological sample suspected of comprising microbes, and isolating nucleic acid sequences from said biological sample;
b) PCR amplifying at least one rRNA internal transcribed spacer (ITS) region comprised in said isolated nucleic acid sequences using a set of broad-taxonomic range amplification primers to thereby generate PCR amplicons;
c) recording a high resolution melting curve for the PCR amplicons generated in step b), and recording the length of the PCR amplicons generated in step b);
d) comparing the high resolution melting curve recorded in step c) with a database comprising high resolution melting curves of reference amplicons generated from reference microbial species or strains of known taxonomic identity using the same set of primers, to thereby obtain a first identity indicator of the microorganism present in said sample;
e) comparing the length of each PCR amplicon having a distinct length recorded in step c) with a database comprising PCR amplicon lengths of reference amplicons generated from reference microbial species or strains of known taxonomic identity using the same set of primers, to thereby obtain a second identity indicator of the microorganism present in said sample;
f) identifying the microorganism present in said sample to the species or strain level if the first and second identity indicator match.

The method of the invention can be used to identify a microorganism present in a sample to the species or strain level, wherein the microorganisms may for instance be a microorganism capable of causing disease or a microorganism that contaminates a product that should be sterile, such as a bacterium, a virus, a protozoa, or a fungus.

If no signal is detected in any of the steps of the method of the invention, it is concluded that the biological sample is negative for the presence of microbes.

The presently claimed method provides for an assay that has a broad scope (i.e. many different micro-organisms can be detected; e.g. universal bacterial), can potentially be performed very fast, is scalable and has a clinically relevant (i.e. very high) sensitivity and specificity because high resolution melting curve (hrMC) analysis, which discriminates on the basis of DNA sequence, and PCR amplicon length analysis, which discriminates on the basis of amplicon length, are used in combination and the confidence on the accuracy of each respective outcome (hrMC characteristics vs. length of amplicon) is interpreted on the basis of the other outcome. While each outcome in isolation may ordinarily not be sufficient to arrive at an unambiguous species designation, or may mistake a contaminating human DNA for microbial DNA, to uniquely find a hrMC outcome to combine or match with a length measurement of an amplicon, provides for an extremely accurate identification of the microbial origin of the amplicons, and thus of the microbe present in the sample. Each outcome is compared to a database comprising hrMC data annotated to microbial strains or species, as well as amplicon length data annotated to microbial strains or species. A microbial species or strain is only then positively identified when both outcomes are annotated to the same species or strains in the database. High resolution melting curves are dependent on length and sequence of an amplicon, but may show overlap between different amplicons as the combination of a differing length with a differing sequence may still lead to comparable melting curve. Combining mrMC with length measurement of amplicons solves this problem. When amplicons have the same melting curve *and* the same length, they must originate from the same microorganism, as amplicons of the same length with the same melting curve must be almost identical in sequence and thus derive from the same species (the sequences of rRNA ITS regions are highly variable between different species, thus small variations can only be found within the same species). Therefore, amplicons that do not derive from the same microorganism, yet show comparable melting curves must have a differing length and conversely amplicons that do not derive from the same species, yet show comparable length must have a differing melting curve. Discrimination of an rRNA ITS amplicon on the basis of melting curve data and amplicon length thereby provides for a highly robust clinical method for microbial identification.

One of the current problems in clinical microbial identification is that human DNA is often co-amplified when microbial amplification primers are employed. It is well-known that the proportion of human DNA in clinical samples may exceed that of bacterial DNA by factors up to 10¹⁰. Although primers for amplifying microbial DNA are specific for microbial DNA, there is always a chance that untargeted human DNA is amplified because of the presence of regions that show homology to parts of the bacterial primers. Even if the chance of such an aspecific amplification is very small (e.g. a 1 in 10⁹ chance), the chance of such an event in an environment where the true target is outnumbered by a factor 10¹⁰ becomes very realistic. Given the high proportion of human DNA in clinical samples, this means that, in principle, in every PCR-based microbial detection assay employing a sample obtained from a human, there is a high probability that false-positive amplification products are formed. When using a classical qPCR approach, this issue is mitigated by employing highly specific probes in addition to amplification primers. These probes can only bind to very specific target regions within amplicons. They will not bind to aspecific products and in this way mitigate the issue of aspecific amplification of human DNA. However, such an approach also prevents the possibility of developing an assay with a broad scope. When aiming to develop a broad scope assay, such aspecific DNA needs to be dealt with in an alternative way. When only using amplicon length analysis, such false-positives cannot be appropriately identified with sufficient clinical certainty, as the length of an aspecific (human) amplicon may overlap with, be similar to or even be identical to that of a micro-organism. With hrMC alone, this problem also remains: aspecific amplicons may produce hrMC curves similar to those of certain microbial strains or species. Again, by combining hrMC with length analysis, this problem can be solved. The combination of hrMC and amplicon length is so specific that an assignment (e.g. being a specific microbial amplicon or an aspecific amplicon) can be made with certainty. When length and hrMC coincide with a microbial signature, the amplicon must be from that micro-organism (as length and hrMC are the same, sequence must be almost identical). If the combination does not coincide with a microbial signature, the amplicon is not of microbial origin. As aspecific human amplicons are generally similar between different patients, a library of hrMC and lengths of human amplicons can be constructed, enabling their straightforward positive identification as being a-specific. By combining broad scope microbial detection with accurate identification of false positives, an assay can be made that can accurately give positive as well as negative results on clinical specimens, both of which results can be used for clinical decision making.

In aspects of the present invention, a database comprising high resolution melting curves and PCR amplicon lengths of reference amplicons generated from reference microbial species or strains of known taxonomic identity as described herein, may further comprise high resolution melting curves and PCR amplicon lengths of reference amplicons generated from human sequences as controls for aspecific amplicon generation using said set of broad-taxonomic range amplification primers. The term "aspecific amplicon generation" must be understood herein as referring to non-microbial, wherein non-specific binding of the primers to a non-target DNA template occurs.

Finally, combining hrMC with amplicon length detection opens up the possibility of miniaturisation of detection devices, paving the way towards Point Of Care (POC) application of a broad scope microbial detection assay. Currently, machinery needed to measure hrMC or amplicon length are typically large and expensive. It is envisioned, however, that these measurements may also be made on very small and relatively cheap devices, such a lab-on-a-chip (LOC) device. The current problem with these devices is that they lack the accuracy of their larger counterparts. An amplicon length may not be measured with an accuracy of 1 nucleotide, but e.g. with 5 nucleotides. For determination of a bacterial species with hrMC or length measurement alone, a high resolution is essential (and even then lacks specificity). However, by combining hrMC information with length measurement, the requirements for resolution of each of these measurements separately may be less stringent. For example, if three different bacterial species were to have 16S-23S ITS amplicon lengths separated by only two nucleotides in length, only a very accurate measurement would be able to differentiate them. However, the fact that these amplicons derive from different bacterial species, implies that the 16S-23S ITS region must be different in sequence (the sequence of the ribosomal DNA and its spacers are unique to every bacterial species). If length is similar, the sequence differences will necessarily lead to very different hrMC profiles, as melting curves are dependent on length and sequence alone. Therefore, even if the three different bacterial species could not be separated by amplicon length alone, they would always be separable by hrMC. As illustrated previously, this is not a matter of chance, as different bacterial species have different sequences of their 16S-23S ITS amplicons. If lengths are similar, then the necessarily differing sequence will lead to different hrMC profiles.

It has now been found that by combining the high resolution melting curve analysis with amplicon length analysis, broad-scope microbial detection and identification for clinical diagnostics becomes feasible. The approach described here can uniquely fulfill all five essential requirements for optimal clinical diagnostics: the scope is very broad, e.g. encompassing all bacterial species; speed is guaranteed as this is a molecular approach and thus negates the need for culture; sensitivity is high as this is a PCR-based approach; specificity of bacterial identification is extremely good by combining hrMC with amplicon length analysis (while, also, specificity from a clinical viewpoint, i.e. correctly identifying true negative samples, is very good by the ability to identify contaminating human DNA); finally, scalability is already good with current machinery and will be further enhanced by miniaturization in the future.

In preferred embodiments of aspects of the invention, the biological sample is a sample from a human body, an animal body, a plant, a food item, a pharmaceutical or chemical product, a laboratory culture or an environmental sample, such as a sample from a soil or water body. In other preferred embodiments, the biological sample is a patient bodily sample selected from a bodily fluid or exudate, including but not limited to uterine fluid, whole blood, serum, plasma, lymph fluid, mucus, saliva, sputum, stool/feces, sweat, wound fluid, pus/purulence, gastric content, ascites/ascitic fluid, bile, urine, semen, cerebrospinal fluid/liquor, and breast milk; or selected from a body sample in the form of a swab, a biopsy, a lavage, or paper point sample, including but not limited to a sample from the skin, an organ, a tissue, the oral cavity, the urogenital tract, the vaginal tract, the gastrointestinal tract, respiratory tract or pulmonary system, and the cardiovascular system.

In preferred embodiments of aspects of the invention, the microorganism is selected from archaea, bacteria, viruses, protozoa, and fungi, preferably said microorganism is a pathogenic microorganism capable of causing disease, preferably selected from pathogenic bacteria, viruses, protozoa, or fungi, most preferably bacteria.

It will be appreciated that the microorganism or microbial nucleic acid sequences in aspects of this invention may be prokaryotic or eukaryotic. One of skill in the art is known with the fact that the internal transcribed spacers (ITS) in the genome of these microorganisms is the spacer DNA situated between the small-subunit ribosomal RNA (rRNA) and large-subunit rRNA genes in the chromosome or the corresponding transcribed region in the polycistronic rRNA precursor transcript. Bacteria and archaea have to ITS regions. The first ITS is located between the 16S and 23S rRNA genes, herein referred to as the 16S-23S rRNA ITS region, the second is located between the 23S and 5S rRNA genes, herein referred to as the 23S-5S rRNA ITS region. There are also two ITS's in eukaryotes; ITS1 is located between 18S and 5.8S rRNA genes and is herein referred to as the 18S-5.8S rRNA ITS region, while ITS2 is located between the 5.8S and 26S (plants) or (28S other eukaryotes) rRNA genes and is herein referred to as the 5.8S-26S/28S rRNA ITS region.

In preferred embodiments of aspects of the invention, at least one rRNA ITS region may therefore be selected from an 16S-23S rRNA ITS region, a 23S-5S rRNA ITS region, an 18S-5.8S rRNA ITS region and a 5.8S-26S/28S rRNA ITS region. In a most preferred embodiment of aspects of the invention, the at least one rRNA ITS region is an 16S-23S rRNA ITS region.

In another preferred embodiment of aspects of the invention, the reference amplicons in the database comprise amplicons generated by an *in vivo* and/or *in silico* PCR amplification reaction for amplifying the corresponding rRNA internal transcribed spacer (ITS) region of a reference microbial species or strain of known taxonomic identity. Preferably the reference microbial species or strain is of the same microbial phylum, more preferably of the same microbial kingdom, most preferably from the kingdom bacteria.

In another preferred embodiment of an aspect of the invention, the databases in step d) and e) are combined into a single database, and wherein said database comprises data on bacteria.

In yet another preferred embodiment of an aspect of the invention, the broad-taxonomic range amplification primers are for amplifying a microbial rRNA ITS region of multiple, preferably essentially all strains or species from a microbial genus, family, order, class, phylum, kingdom and/or domain, preferably essentially all strains or species from a microbial phylum, more preferably essentially all strains or species from a microbial kingdom, most preferably bacteria.

The term "essentially all strains or species", as used herein, preferably refers to a situation wherein more than 50%, preferably more than 60%, 70%, 80%, 90% or more of the microbial strains or species comprised in said genus, family, order, class, phylum, kingdom and/or domain, are amplified by said broad-taxonomic range amplification primers.

In still another preferred embodiment of an aspect of the present invention, the broad-taxonomic range amplification primers for amplifying at least one rRNA internal transcribed spacer (ITS) region comprise a forward and reverse primer for amplifying a 16S-23S rRNA ITS region, a 23S-5S rRNA ITS region, an 18S-5.8S rRNA ITS region or a 5.8S-26S/28S rRNA ITS region. In a most preferred embodiment of aspects of the invention, the broad-taxonomic range amplification primers for amplifying the at least one rRNA internal transcribed spacer (ITS) regions comprise a forward and reverse primer for amplifying a 16S-23S rRNA ITS region.

The term "at least one microbial rRNA ITS region", as used herein refers to the embodiment wherein a microbial rRNA ITS region is amplified over its entire length. One of skill will readily understand that broad-taxonomic range amplification of microbial rRNA ITS regions requires the presence of identical sequences (or sequences having a high level of sequence similarity, e.g. more than 90%, preferably more than 95%, 96%, 97%, 98%, or 99% sequence similarity over the entire length of the sequence) in the genomic DNA of a large number of taxonomically related strains or species to function as primer annealing sites. Such identical sequences or sequences with a high level of sequence similarity are usually found in conserved regions of the coding rRNA sequence, and not in the ITS regions. Therefore, amplifying at least one microbial rRNA ITS region by broad-taxonomic range amplification of microbial rRNA ITS regions will usually require identification of a conserved region in opposite coding rRNA regions of the rRNA ITS of interest. As a result, the ITS region is usually amplified in its entirety. One of skill in the art will understand that the subsequent annotation of the taxonomic identity may occur by considering only a part of the microbial rRNA ITS of interest or a part of the PCR amplicon generated in methods of this invention, although it is foreseen that this preferably occurs by considering the microbial rRNA ITS as amplified in its entirety.

In yet another preferred embodiment of an aspect of the invention, the set of broad-taxonomic range amplification primers comprises each of the amplification primers of SEQ ID NOs: 1 and 3-5, or each of the amplification primers of SEQ ID NOs: 2-5, or each of the amplification primers of SEQ ID NOs: 1-5.

In yet another preferred embodiment of an aspect of the invention, the set of broad-taxonomic range amplification primers comprises each of the amplification primers of SEQ ID NOs: 6 and 7-13.

In yet another preferred embodiment of an aspect of the invention, the set of broad-taxonomic range amplification primers is a set of universal bacterial amplification primers, said set preferably comprising each of the amplification primers of SEQ ID NOs: 14-15.

In yet another preferred embodiment of an aspect of the invention, the step of PCR amplifying comprises qPCR.

In yet another preferred embodiment of an aspect of the invention, the length of the PCR amplicons is recorded by capillary electrophoresis.

In yet another preferred embodiment of an aspect of the invention, the PCR amplification reaction further comprises a PCR calibrator system, comprising a set of PCR amplification primers at least one of which primers comprises a label, and a set of at least two PCR calibrators, each PCR calibrator consisting of a DNA fragment comprising a spacer region having a DNA sequence of a given length flanked by upstream and downstream adapter DNA sequences that comprise primer binding sites for binding of said PCR amplification primers wherein said set of PCR amplification primers is for PCR amplifying the spacer region DNA sequence of all PCR calibrators in said set of at least two PCR calibrators, wherein the spacer region DNA sequence comprised in each of said PCR calibrators in said set of at least two PCR calibrators is of a different length, and wherein each PCR calibrator in said set of at least two PCR calibrators is present in equal amount or in a known amount relative to other PCR calibrators in said set; and wherein said step b) of PCR amplifying further comprises PCR amplifying the at least two PCR calibrators using the PCR amplification primers of the PCR calibrator system.

In still another preferred embodiment of an aspect of the present invention, the broad-taxonomic range amplification primers for amplifying the at least one rRNA internal transcribed spacer (ITS) regions comprise a labelled forward and/or labelled reverse primer, preferably a labelled forward primer, more preferably a fluorescently labelled forward primer.

### DESCRIPTION OF THE DRAWINGS

Figure 1 Cycling schedule including melting curve analysis as performed on the LightCycler480 machine. Fluorescence acquisition was performed at the end of the primer extension phase at 72°C (dots) and during the entire melt indicated by the temperature gradient starting at 1:43:17.
Figure 2. Example melting curve analysis. Top panel: A decrease of fluorescence can be found at increasing temperatures, as double stranded DNA increasingly becomes single stranded and EvaGreen loses its fluorescence. Lower panel: A first derivative of the top melting curve clearly accentuates areas of sudden decrease in fluorescence: the melting temperatures of target amplicons.
Figure 3. Melting curves (left column) and fragment length analyses of 16S-23S fragments of eight different bacterial species from three different phyla.
Figure 4. 16S-23S fragment lengths of *Streptococcus pyogenes* and *Corynebacterium jeikeium.* Both species show identical length profiles. However, the melting curve of each species is unique. By combining length data with melting curve data, both species can be unambiguously identified.
Figure 5 Combined fragment length analysis and melting curve analysis of 16S-23S IS profiles of *Klebsiella pneumoniae* and *Enterobacter cloacae.* While melting curves are almost impossible to discriminate, each species can be identified by a unique fragment length signature.

### DETAILED DESCRIPTION OF THE INVENTION

### Definitions

The term "nucleic acid sequence", or "nucleotide sequence", which terms can be used interchangeably herein, refers to the base sequence of a DNA or RNA molecule in single or double stranded form, particularly a DNA sequence encoding an ITS region of the ribosomal RNA gene.

An "isolated nucleic acid sequence" refers to a nucleic acid sequence which is no longer in the natural environment from which it was isolated. The term *inter alia* refers to a nucleic acid molecule that has been separated from at least about 50%, 75%, 90%, or more of proteins, lipids, carbohydrates, or other materials with which it is naturally associated, e.g. in a microbial host cell.

The term "microbial", as used herein, refers to a subject as originating from a microorganism, or microbe, which generally refers to an organism that is microscopic, which means too small to be seen by the unaided human eye.

The term "target microbial nucleic acid sequence" , as used herein, refers to the nucleic acid fragment targeted for replication (or amplification) and subsequent detection and that is diagnostic of a particular microorganism whose presence is to be determined.

The term "polymerase chain reaction (PCR)" as used herein refers to the well known *in vitro* technique to make numerous copies of a specific segment of target DNA from a template DNA - i.e., the DNA that contains the target region to be copied. During the reaction a mixture containing the target DNA, primers, dNTPs, and a heat-stable DNA polymerase is heated to 90-95°C to denature the strands of the target DNA. The solution is cooled to a temperature that allows the primers (single-stranded DNA molecules of about 18 to 30 nucleotides long) to anneal to their complementary sequence on the target DNA and provide the 3'-OH required for DNA synthesis. Subsequently, the DNA polymerase synthesizes a new DNA strand complementary to the target by extending the primer, usually at a temperature of about 72°C. The thermal cycling scheme of denaturing/primer annealing/ primer extension is repeated numerous times with the DNA synthesized during the previous cycles serving as a template for each subsequent cycle. The result is a doubling of the target DNA present with each cycle, and exponential accumulation of target DNA sequences over the course of 20-40 cycles. A heating block with an automatic thermal cycler is used for precise temperature control. A preferred method for use in the present invention is qPCR amplification (also known as real-time PCR), wherein typically the amplification of a targeted DNA molecule is monitored during the PCR (i.e., in real time), using non-specific fluorescent dyes that intercalate with any double-stranded DNA or sequence-specific DNA probes consisting of oligonucleotides that are labelled with a fluorescent reporter for the detection of PCR products in real-time.

The term "template", as used herein, refers to the nucleic acid from which the target sequence is amplified in a nucleic acid amplification reaction. The term "amplifiable template", as used herein, refers to a template that, when amplified, results in a single amplicon. Amplifiable templates comprise primer binding sites for hybridization of amplification primers.

The term "isolating", as used herein in the context of isolating nucleic acid sequences from a biological sample, refers to an *in vitro* process wherein nucleic acids, preferably genomic DNA, are extracted from a sample of interest. The process may generally involve, but is not limited to, lysis of a (cells in) biological sample using a guanidine-detergent lysing solution that permits selective precipitation of DNA from a (cell) lysate, and precipitation of the genomic DNA from the lysate with ethanol. Following an ethanol wash, precipitated DNA may be solubilized in either water or 8 mM NaOH and used as template in a PCR reaction. Genomic DNA samples analysis with diagnostic purpose may be obtained by using generally known techniques for DNA isolation. The total genomic DNA may be purified by using, for instance, a combination of physical and chemical methods. Very suitably commercially available systems for DNA isolation may be used, such as the NucliSENS^{®} easyMAG^{®} nucleic acid extraction system (bioMérieux, Marcy l'Etoile, France) or the MagNA Pure 96 System (Roche Diagnostics GmbH, Mannheim, Germany).

The term "PCR mixture", as used herein, refers to the small volume of biochemical reactants in aqueous liquid for performing the PCR reaction comprising the (genomic) template DNA comprising the target DNA sequence(s), a set of at least two oligonucleotide primers that hybridize to opposite strands of the target DNA sequence(s) and flank the region to be amplified, a thermo-stable DNA polymerase, the four deoxyribonucleoside triphosphates (dNTPs), and Mg2+ ions.

The term "amplification primers", as used herein, refers to the oligonucleotide primers that hybridize to opposite strands of the target DNA sequence(s) and flank the region to be amplified.

The terms "amplification product", and "amplicon", as used interchangeably herein, refer to a nucleic acid fragment that is the product of a nucleic acid amplification or replication event, such as for instance formed in the polymerase chain reaction (PCR). The term "PCR amplicon", as used herein, refers to the PCR product or amplified target DNA.

The term "high resolution melting curve (hrMC) analysis", as used herein, refers to a post-PCR analysis method used to identify variations in nucleic acid sequences. The method is based on detecting small differences in PCR melting (dissociation) curves. The temperature-dependent dissociation between two DNA-strands can be measured using a DNA-intercalating fluorophore such as SYBR green, EvaGreen or a "saturation dye" (a dye that does not inhibit PCR even if used at concentrations that give maximum fluorescence (saturation)) like LCGreen^{®} I, LCGreen Plus or Cyto9, in conjunction with real-time PCR instrumentation that has precise temperature ramp control and advanced (fluorescence) data capture capabilities. Data are analyzed and manipulated using software designed specifically for hrMC analysis. High resolution melt curves are generated by slowly ramping through a temperature gradient with a high level of accuracy (e.g. 0.1 °C or less), and measuring the level of fluorescence of an intercalating dye at each step. The melting temperature of a DNA molecule is determined by nucleic acid sequence and length, and differences in these nucleotide sequences between samples result in melting profiles that are unique to a particular species, even when amplicons are isolated using universal primers. Details of the hrMC analysis procedure are well known to those skilled in the art and are for instance described in Reed GH, Kent JO, Wittwer CT (2007) High-resolution DNA melting analysis for simple and efficient molecular diagnostics. Pharmacogenomics,8, 597-608; US7,297,484; US7,387,887; US7,524,632; US20090117553 and US20100041044.

The term "high resolution melting curve (hrMC)", as used herein, refers to the dissociation curve describing the temperature-dependent dissociation between two DNA-strands as measured using a DNA-intercalating fluorophore. The high resolution melting curve may refer to the graph of the negative first derivative of the melting-curve which makes it easier to pin-point the temperature of dissociation (defined as 50% dissociation), by virtue of the peaks thus formed.

The term "electrophoretic separation and amplicon length analysis", as used herein, refers to the technique whereby mixtures of charged molecules, preferably nucleic acids, in particular PCR amplified DNA fragments, loaded on a gel matrix are caused to migrate from the negative electrode (cathode) toward the positive electrode (anode), on the basis of size, charge, and structure, through the gel when said gel is placed in an electrical field, whereby shorter nucleic acid fragments migrate more rapidly than longer ones, resulting in separation based on size. Electrophoretic separation and amplicon length analysis is preferably performed by capillary electrophoresis, whereby the DNA is detected either by UV absorption or by fluorescent labeling. In the presence of appropriate standards, fragments can be accurately sized based on relative electrophoretic mobility, for instance using a ABI Prism 3500 Genetic Analyzer (Applied Biosystems) or similar analyzers.

"Electrophoretic separation and amplicon length analysis", as defined herein, may also be performed by DNA sequencing of the amplicons. DNA sequencing may provide accurate information on the length of the amplicon.

The term "negative control reaction", as used herein, refers to a post-PCR mixture comprising no PCR amplicon(s) as a result of the deliberate absence of target nucleic acid sequences or template DNA in the pre-PCR mixture.

The term "primer dimer (PD)", as used herein, refers to a potential by-product in PCR, consisting of primer molecules that have annealed (hybridized) to each other because of strings of complementary bases in the primers. As a result, the DNA polymerase amplifies the PD, leading to competition for PCR reagents, thus potentially inhibiting amplification of the DNA sequence targeted for PCR amplification. In quantitative PCR, PDs may interfere with accurate quantification.

The term "non-specific PCR amplicon", as used herein, refers to a potential by-product in PCR, consisting of amplified DNA that is not target DNA, usually resulting from a-specific annealed (hybridization) of the primer molecules to other nucleic acid sequences in the template DNA, such as human DNA. A non-specific PCR amplicon results in a hrMC that is different from the PCR amplicon generated from a target microbial DNA sequence.

The term "human PCR amplicon", as used herein, refers to a non-specific PCR amplicon whereby primer molecules have annealed to human nucleic acid sequences in the template DNA instead of microbial DNA sequences. A human PCR amplicon results in a hrMC that is different from the PCR amplicon generated from the (microbial) target DNA.

The term "quantification cycle" or "Cq" as used herein includes reference to a measurement taken in a real time PCR assay or qPCR assay, whereby a positive reaction is detected by accumulation of a signal, such as a fluorescent signal. The Cq (quantification cycle) can be defined as the number of cycles required for the signal to cross the threshold (i.e. exceeds background level). Cq levels are inversely proportional to the amount of target nucleic acid in the sample (i.e. the lower the Cq level the greater the amount of target nucleic acid in the sample).

The term "sample" or "biological sample", as used herein, includes reference to a sample from a human body, an animal body, a plant, a laboratory culture, an environmental sample or a food item, a pharmaceutical or chemical product, preferably wherein said food item, pharmaceutical or chemical product is intended to be devoid of microbes or microbial DNA.

The term "subject", as used herein is intended to refer to any individual or patient to which the method described herein is performed. Generally the subject is human, although as will be appreciated by those in the art, the subject may be an animal. Thus other animals, including mammals and birds are included within the definition of subject.

### Performing PCR in a method of the invention

In a method of the invention, a step of amplifying DNA is performed by the polymerase chain reaction (PCR).

Preferably, in a method of the invention, PCR is a qPCR or real-time PCR. The present invention relates to broad-taxonomic range amplification or universal amplification of at least one rRNA ITS region of microbial genomic DNA in a sample, i.e. a taxon-specific amplification of genomic DNA of a whole microbial genus, family, order, class, phylum or kingdom.

The skilled person is well aware of methods and means for setting up such a PCR reaction. Preferably, a method of the invention is for detecting or identifying a bacterium or bacterial DNA in a sample. Exemplary primer sets for amplifying a 16S-23S rRNA ITS region from genomic DNA of one or more of the bacterial phyla *Firmicutes*, *Bacteriodetes* or *Proteobacteria* are provided as SEQ ID NOs:1-13. An exemplary primer set for universally amplifying a 16S-23S rRNA ITS region from genomic DNA of bacteria is provided as SEQ ID NOs: 14-15. It is within the routine capabilities of the skilled person to design further primer sets that allow for broad-taxonomic range amplification of microbial, preferably bacterial, DNA.

### High resolution melting curve (hrMC) analysis in a method of the invention

Nucleic acid characterization by high resolution Melting Curve (hrMC) analysis is a powerful technique for identifying sequence variation during PCR or in a post-PCR sample. By measuring the fluorescence of a saturating intercalating dye as PCR-amplified DNA fragments are heated and disassociate, sequence-defined melt curves can be generated with single-nucleotide resolution.

In preferred embodiments of aspects of this invention, the hrMC dye is selected from the group consisting of, but not limited to, LC Green, SYTO9, Eva Green, Chromofy, BEBO, or SYBR Green, preferably Eva Green. The saturating intercalating dye should preferably not inhibit PCR.

In a method of the invention, a hrMC profile for the PCR amplicons may be generated during the step of PCR amplification, or after the step of PCR amplification, by measuring the fluorescence of a saturating intercalating dye of amplicons present in a post-PCR sample. As used herein, a "melting profile" refers to a profile generated by hrMC analysis. One of skill in the art is well aware how hrMC is to be performed. Ample guidance can be found, for instance in Reed et al. 2007 Pharmacogenomics 8(6): 597-608 and Wittwer et al. 2003 Clin Chem. 49:853-860.

High resolution melting curve analysis in qPCR (i.e. real-time PCR) is not equivalent to that performed by digital PCR. In digital PCR, each partition contains only a single sample template, which means that all amplicons for each partition originate from a single template. This homogeneity allows for easier interpretation of hrMC profiles and allows for direct comparison with other partitions. In real-time PCR, the heterogeneous sample of multiple targets after amplification will still remain heterogeneous and thus the melting curve will be a reflection of that heterogeneity. This makes discriminating multiple targets via hrMC analysis difficult in case that multiple amplicons are present in sample.

In a method of the present invention, a hrMC profile of a post-PCR sample is generated. Preferably, the PCR is a qPCR or real-time PCR. The generated hrMC profile is compared to (predetermined) reference hrMC profiles of one or more corresponding 16S-23S rRNA ITS amplicons of known (i.e., taxonomically identified) strains or species of microbes. Preferably, said reference hrMC profiles of one or more corresponding 16S-23S rRNA ITS amplicons of known strains or species is comprised in a library or database of reference hrMC profiles. Such a library or database can be established by (i) *in vitro* generating 16S-23S rRNA ITS PCR amplicons of individual strains and/or species of microbes, (ii) performing an hrMC analysis of said amplicons, and (iii) storing said reference hrMC profiles in a library or database of reference hrMC profiles, wherein the species or strain identity is annotated to the hrMC profile. Alternatively, a database or library of reference hrMC profiles can be generated by *in silico* prediction of an hrMC profile of one or more 16S-23S rRNA ITS PCR amplicons of individual strains and/or species of microbes.

From a clinical perspective, if a hrMC profile of a 16S-23S rRNA ITS PCR amplicon in a PCR sample indicates that a microbe, such as a bacterium, is present in said clinical sample, the clinician can already make an important first treatment decision: start administering a therapeutic amount of an anti-microbial agent if a microbe is present that is sensitive to said anti-microbial agent. For instance, if it is determined on the basis of hrMC analysis that a bacterium is present, the clinician can make the treatment decision of starting administration of an antibiotic.

However, in many instances qPCR or real-time PCR does not lead to definitive identification of the microbe present. hrMC analysis of the PCR product can provide a definitive answer as to the identity of the microbe if there is a match with a reference hrMC profile only in the case that a single microbe is present in a sample. In other cases, such as when more than one microbe is present in said sample, hrMC analysis generally does not provide actionable information to the clinician regarding the identity of the microbe(s).

The present inventor has discovered that it is highly beneficial in terms of increasing sensitivity and specificity of PCR-based microbial identification assays to combine in one clinical assay sequence-discriminating hrMC analysis with PCR fragment length analysis which discriminates on the basis of fragment length. This combinatorial approach allows for interpretation of the generated hrMC profile on the basis of a PCR fragment length profile, and vice versa, which greatly increases specificity. This was hitherto unknown in the art. Combining the two techniques provides the clinician with actionable information on microbe identity. Preferably, the specificity (of microbial identification) provided by a method of the invention is preferably at least 90%, more preferably at least 91%, 92%, 93%, 94%, 95%, 96%, 97%, 98% or at least 99%.

### PCR fragment length analysis in a method of the invention

A method of the invention involves a step of amplicon fragment length analysis performed on amplicons in a PCR sample in order to generate a fragment length profile of the amplicons present therein.

Methods and means for determining (PCR) fragment length profiles are generally known in the art. One exemplary and highly beneficial method for PCR fragment length analysis is capillary gel electrophoresis. Preferably, the capillary gel electrophoresis method is a capillary gel electrophoresis method referred to as the IS-pro method as described in Budding et al., FASEB J., 24, 4556-4564 (2010), WO2008/125365 and WO2015/170979 and may, in preferred embodiments, involve bacterial species differentiation on the basis of the length of the 16S-23S rDNA ITS region with taxonomic classification by phylum-specific fluorescent labelling of PCR primers.

The IS-pro method is described in the aforementioned publication. For instance, a method for analysis of populations of micro-organisms is described on for instance page 4, lines 11 and further of WO2008/125365, including primer sets. In addition, for instance, the PCR calibrator system and its use is described in WO 2015/170979 (see e.g. p.44 and further), the IS-pro analysis as described in WO 2015/170979 (see e.g. p.53 and further), primer sets and probes as described in WO 2015/170979 (see e.g. 65 and 66 of WO 2015/170979), and types of patient samples as described in WO 2015/170979 (see e.g. p. 11, line 18 and further).

In short, with regard to the IS-pro method, the sequences of conserved DNA regions comprised in the 16S and 23S rRNA gene sequences flanking the intergenic region in the genomic DNA of the microorganism are used as primer binding sites for amplification of the (polymorphic) ITS DNA region.

The taxonomic diversity analysis of IS-pro is based on the fact that prokaryotic microorganisms, including bacteria and archaea, comprise in their genome one or more copies of the rrn operon comprising the genes for the 5S, 16S and 23S ribosomal RNAs. In most prokaryotes the ribosomal genes in the operon are in the order 16S-23S-5S and are co-transcribed in a single polycistronic RNA that is processed to provide the RNA species present in the mature ribosome. The spacer between the 16S and 23S genes contains regions with secondary structures and sometimes tRNA genes. The variation in the spacers of the rRNA operons found among relatively close taxa is very high. The extreme divergence in size and sequence of the spacers among different groups of prokaryotes makes them ideally suited as taxonomic markers.

In the IS-pro method, the 16S-23S rRNA intergenic spacer (ITS) region is amplified using primers directed to conserved regions in the ribosomal gene sequences. More preferably, the conserved DNA regions are those located nearest to the 3'-end of the 16S rRNA gene and nearest to the 5'-end of the 23S rRNA gene.

Depending on the microbiome investigated, phylum-specific primer sets may be used. Suitable phylum-specific primer sets in IS-pro include such primer sets that allow simultaneously amplification of multiple sequences in a single reaction in a process referred to as multiplex PCR.

For amplification of intergenic spacer region of bacterial DNA from the phyla *Firmicuta* and *Actinobacteria* a suitable primer set includes:
FirISf: 5'-CTGGATCACCTCCTTTCTAWG-3' (SEQ ID NO: 1) as the forward primer and one of DUISrI : 5' -AGGCATCCACCGTGCGCCCT-3' (SEQ ID NO:3), DUISr2 : 5' -AGGCATTCACCRTGCGCCCT-3' (SEQ ID NO:4) and DUISr3: 5'-AGGCATCCRCCATGCGCCCT-3' (SEQ ID NO:5) as the reverse primer. Preferably herein, the FirISf primer is labeled with a fluorescent label. Preferably herein, the reverse primers are non-labeled.

Genera within the phylum *Firmicutes* include: *Bacilli*, order *Bacillales* (*Bacillus*, *Listeria*, *Staphylococcus*); *Bacilli*, order *Lactobacillales (Enterococcus, Lactobacillus, Lactococcus, Leuconostoc, Pectinatus*, *Pediococcus, Streptococcus); Clostridia (Acetobacterium, Clostridium, Eubacterium*, *Heliobacterium*, *Heliospirillum*, *Sporomusa*); *Mollicutes (Mycoplasma, Spiroplasma*, *Ureaplasma*, *Erysipelothrix).*

For amplification of intergenic spacer region of bacteria from the phylum *Bacteroidetes* a suitable primer set includes:
BacISf: 5' -CTGGAACACCTCCTTTCTGGA-3' (SEQ ID NO:2) as the forward primer and one or more, preferably all, of DUISrI : 5' - AGGCATCCACCGTGCGCCCT-3' (SEQ ID NO:3), DUISr2 : 5' - AGGCATTCACCRTGCGCCCT-3' (SEQ ID NO:4) and DUISr3: 5'-AGGCATCCRCCATGCGCCCT-3' (SEQ ID NO:5) as the reverse primer. Preferably herein, the BacISf primer is labeled with a fluorescent label. Preferably herein, the reverse primers are non-labeled.

In broad community analysis, the intergenic spacer region of bacteria from the phyla *Firmicuta* and *Actinobacteria* can be amplified in a single multiplex reaction with the intergenic spacer region of bacteria from the phylum *Bacteroides.* In such a multiplex reaction, both forward primers FirISf and BacISf as well as all three reverse primers are included in the PCR mixture. Preferably herein, the FirISf primer is labeled with a first fluorescent label, e.g. FAM, and the BacISf primer is labeled with a second fluorescent label, e.g. HEX. Preferably herein, the reverse primers are non-labeled.

For still broader, or alternative community analysis, the intergenic spacer region of bacteria from the phylum *Proteobacteria* may be amplified, a suitable primer set for which includes:
ProtISf: 5'-CCGCCCGTCACACCATGG-3' (SEQ ID NO:6) as the forward primer and one or more, preferably all, of DPISr1: 5'-AATCTCGGTTGATTTCTTTTCCT-3' (SEQ ID NO:7), DPISr2: 5'-AATCTCGGTTGATTTCTTCTCCT-3' (SEQ ID NO:8), DPISr3: 5'-AATCTCTTTTGATTTCTTTTCCTCG-3' (SEQ ID NO:9), DPISr4: 5'-AATCTCATTTGATGTCTTTTCCTCG-3' (SEQ ID NO:10), DPISr5: 5'-AATCTCTTTTGATTTCTTTTCCTTCG-3' (SEQ ID NO:11), DPISr6: 5'-AATCTCTCTTGATTTCTTTTCCTTCG-3' (SEQ ID NO:12), and DPISr7: 5'-AATCTCAATTGATTTCTTTTCCTAAGG-3' (SEQ ID NO:13) as the reverse primer. Preferably herein, the ProtISf primer is labeled with a fluorescent label. Preferably herein, the reverse primers are non-labeled.

Genera within the phylum *Bacteroidetes* include the genera *Bacteroides* (an abundant organism in the feces of warm-blooded animals including humans; including for example *B. acidifaciens*, *B. distasonis*, *B. gracilis*, *B. fragilis*, *B. oris*, *B. ovatus*, *B. putredinis*, *B. pyogenes*, *B. stercoris*, *B. suis*, *B. tectus*, *B. thetaiotaomicron*, *B. vulgatus*), and *Porphyromonas*, a group of organisms inhabiting the human oral cavity.

The amplification of the phylum of *Proteobacteria* is very beneficially performed by carrying out a multiplex PCR in order to provide sufficient taxonomic resolution within the proteobacterial phylum (see WO 2015/170979).

Preferred microorganisms in aspects of this invention belong to bacterial phyla selected from the group consisting of *Firmicutes*, *Fusobacterium*, *Deferribacteres*, *Spirochaetes*, *Cyanobacteria*, *Acidobacteria*, *Nitrospina*, *Nitrospirae*, *Caldithrix*, *Haloanaerobiales*, *Verrrucomicrobia*, *Chlamydiae*, *Planctomycetes*, *Gemmimonas*, *Fibrobacteres*, *Chlorobi*, *Bacteroidetes*, *Proteobacteria*, *Thermotogae*, *Corprothermobacter*, *Synergites, Thermodesulfobacteria*, *Desulfurobacterium*, *Aquificae*, *Deinococcus-Thermus*, *Chloroflexi*, *Tenericutes* and *Actinobacteria.* More preferred phyla targeted in methods of this invention comprise *Bacteroidetes*, *Firmicutes*, *Actinobacteria*, *Proteobacteria*, *Fusobacteria* and *Verrrucomicrobia.* Highly preferred are *Bacteroidetes*, *Firmicutes*, *Actinobacteria* and *Proteobacteria.*

These phyla are known to the person skilled in the art and have been described, *inter alia*, in Schloss 2004 (Microb. Mol. Biol. Rev. 6 (4): 686-691), or in the Bergey manual of systematics of archaea and bacteria (2015).

Also a universal amplification of bacterial 16S-23S rRNA internal transcribed spacer (ITS) region of genomic DNA can be employed in a method of the invention. For such a universal amplification of bacterial DNA, one can employ a universal bacterial primer set comprising forward primer ITS1FD having the sequence 5' - CGGTGAATACGTTCCCGGIIIIIGTACAC -3' (SEQ ID NO: 14) in combination with reverse primer ITS2RD having the sequence 5'-CGTCCTTCDTCGVCTBIIIIIGCCARG-3' (SEQ ID NO: 15).

The samples on which a method of the present invention may be performed may be from any environment comprising microorganisms, but are preferably samples from mammals such as humans. Suitable sample materials include for example samples from humans, plants, animals, water, food (like dairy products), yeast cultures (e.g. used in industry) or soil.

In the IS-pro technique, microbial DNA is suitably, and generally, isolated by an automated isolation procedure (e.g. EasyMag, Biomerieux, Marcy l'Etoile, France) according to the manufacturer's instructions. In a method of the present invention, microbial DNA is preferably isolated from a sample in the form of genomic DNA. One of skill is well aware of genomic DNA isolation methods available in the art.

Amplification of 16S-23S rRNA ITS regions with phylum-specific fluorescently labeled PCR primers from isolated microbial DNA is then performed with the IS-pro assay (inBiome, Amsterdam, the Netherlands) according to the protocol provided by the manufacturer, or by using any of the primer sets described above. IS-pro involves amplification of 16S-23S rRNA ITS regions and subsequent identification of the microbial species as the source of the DNA template from which the amplicon is generated based on the length of the amplicon. In short, appropriately diluted isolated DNA can be amplified in a standardized PCR amplification. One example of a standardized PCR amplification includes the use of phylum-specific primer sets for amplification of the 16S-23S rRNA ITS region of the combined phyla of *Firmicutes*, *Actinobacteria*, *Fusobacteria*, and *Verrucomicrobia* (FAFV). Another example of a standardized PCR amplification includes the use of phylum-specific primer sets for amplification of the 16S-23S rRNA ITS region of the phylum *Bacteroidetes.* Also, the 16S-23S rRNA ITS region of the phyla *Firmicutes*, and *Bacteroidetes* may be amplified in a single reaction to provide amplicons from any bacterial species from these phyla from a DNA sample (for instance using the primers of SEQ ID NO: 1-5 in a single multiplex reaction). Yet another example of a standardized PCR amplification includes the use of phylum-specific primer sets for amplification of the 16S-23S rRNA ITS region of the phylum *Proteobacteria* (for instance using the primers of SEQ ID NO:6 and 7-13). Still another example of a standardized PCR amplification includes the use of kingdom specific primer sets for amplification of the 16S-23S rRNA ITS region of microorganisms belonging to the kingdom *Bacteria* (for instance using the primers of SEQ ID NO: 14 and 15).

Following PCR amplification and generation of amplicons, a small aliquot of the PCR product may then be separated and analysed by capillary electrophoresis to reveal the 16S-23S rRNA ITS amplicon length, for instance in an ABI Prism 3500 Genetic Fragment Analyzer (Applied Biosystems Carlsbad, California, USA).

In IS-pro, distinct bacterial species having distinct 16S-23S ITS DNA sequences generate amplicons of different length when using broad-taxonomic range or universal amplification primers for amplifying 16S-23S rRNA ITS region from a microbial genomic DNA template in a sample, which amplicons are readily separated by capillary gel electrophoresis, thereby providing a profile of distinct DNA fragments, each representing a separate 16S-23S ITS DNA sequence having a characteristic length (in number of nucleotides or base pairs).

Generally, each fragment is considered to represent a single ITS region from a distinct bacterial species or operational taxonomic unit (OTU). However, it is possible that different bacterial species generate a 16S-23S ITS amplicon of identical length. Moreover, since genomes may comprise multiple rrn operons with different ITS region sequences, not every fragment necessarily represents a distinct microbial species. Hence, different peaks (amplicons of distinct length) may originate from a single species or from distinct species. Finally, small biological length variations in the ITS sequence may complicate straightforward species identification based on length measurement alone.

In order to improve resolution and species identification and to better classify, and assign to a particular species or strain, amplicons from a microbial DNA sample, the present inventor has now found that adding the step of hrMC analysis may result in the separate identification of the individual species that generate amplicons of identical length (i.e. that give a single electrophoresis peak). The hrMC analysis enables identification of sequence differences between amplicons of identical length and, hence, allows for individual identification of the species that underlie the same CE peak. Moreover, multiple different length amplicons originating from a single species may be unambiguously assigned to the correct species by the addition of the hrMC step. Also, fragments that may in combination originate from either a single species or from (multiple) distinct species, may be classified and assigned to a either originating from particular species or strain, or originating from multiple species or strains. This can be done either in complex and non-complex microbial populations alike.

These improvements are extremely important for reliability of an assay for use in clinical practice.

In addition, it has now been found that certain amplicon peaks in the amplicon length profile can now be attributed to human DNA (a false-positive) which greatly increases specificity of the assay in the clinic because ambiguous length peaks in the amplicon length profile that cannot be dismissed by the clinician as "non-microbial" on the basis of an amplicon length profile, can now in fact be dismissed if the hrMC analysis provides confirmation that human DNA indeed is present (identification of a false positive).

The specificity of such tests is improved as the hrMC of distinct microbial species is generally easily distinguishable, leading to unambiguous detection of individual species that produce identical amplicon lengths in PCR (e.g. *C. jeikeium* / *S. pyogenes*) based on the difference in the hrMC of their amplicon.

A method of the present invention includes the step of adding a hrMC dye prior to, during or after PCR amplification, and performing hrMC analysis on the post-PCR mixture, in particular the PCR amplicon(s).

It is another advantage of the use of hrMC analysis in diagnostic methods involving amplification reactions with broad-taxonomic range primers and subsequent electrophoretic separation and amplicon length analysis, that the hrMC analysis can add in the speed of the procedure. Once the hrMC of a reaction product is known, one may perform the electrophoretic separation and amplicon length analysis for a predetermined length range of fragments only, based on the hrMC data (e.g. only separating and analyzing fragments in length between 300-500 bp). For instance, if the hrMC indicates the presence of one of three possible species, one may immediately "zoom in" on the amplicon lengths known for those species to resolve the matter.

Methods of the present invention may be used to type, diagnose, investigate, analyse and monitor such diverse microbe harbouring samples as those associated with the gut or gastrointestinal tract, the skin, the urogenital tract, the oral cavity, and the pulmonary system, and diagnose, monitor or predict disease. The methods may be used for diagnostic purpose such as for diagnosing, monitoring or predicting (incl. early detection of) a microbial infection, and may even be used for environmental diagnostics, such as for determining the microbial status of a sample source, wherein said sample source is of environmental, plant, animal or food origin, or a sample of a pharmaceutical or chemical product intended to be devoid of microbes or microbial DNA and wherein specific profiles of ITS regions (or the absence thereof) are indicative of sterility of said sample, quality of the environment, microbial safety of a food, pharmaceutical product, the quality of a chemical product or the health of plant or animal.

An alternative method for determining a PCR amplicon length is by a step of DNA sequencing performed on amplicons in said post-PCR sample. Preferably, said step of DNA sequencing is performed by next-generation sequencing methods that are capable of determining a sequence length of amplicons having a length in base pairs in the range of 100-1200 bps, which range generally resembles the overall variation in length of the 16S-23S ITS DNA sequence between different microbial species. Suitable next-generation sequencing methods to determine amplicon length include nanopore-based approaches (e.g. devices manufactured by Oxford Nanopore) or Pacific-Biosciences or single molecule real-time sequencing approaches (e.g. devices manufactured by Pacific Biosciences).

When a step of determining PCR amplicon length is performed in a method of the invention, an amplicon length profile can be generated. Such a generated amplicon length profile can be compared to one or more reference or control amplicon length profiles of a corresponding 16S-23S rRNA internal transcribed spacer (ITS) region of a known strain or species of microbe. Preferably, said reference amplicon length profile of a corresponding 16S-23S rRNA internal transcribed spacer (ITS) region of a known strain or species of microbe is comprised in a library or database of reference amplicon length profiles. Such a library or database can be established by (i) *in vitro* generating PCR amplicons of a target 16S-23S rRNA internal transcribed spacer (ITS) region of genomic DNA of known individual strains and/or species of microbes, (ii) performing a fragment length analysis of said amplicons, and (iii) storing said reference fragment length profiles in a library or database of reference fragment length profiles. Alternatively, a database or library of reference fragment length profiles can be generated by *in silico* prediction of a fragment length profile of PCR amplicons of a target 16S-23S rRNA internal transcribed spacer (ITS) region of genomic DNA of individual strains and/or species of microbes.

It is within the metes and bounds of routine experimentation of the skilled person to establish (pre-determined) reference or control amplicon length profiles suitable for comparison with the generated amplicon length profile of the test sample. For instance a method of the invention may include the step of analyzing amplification products or amplicons (in a post-PCR) based on length differences in said amplification products to thereby provide an amplicon length profile of the composition of a population of microorganisms in a microbiome or a sample as disclosed herein; and comparing said fragment length profile with at least one reference fragment length profile of a known micro-organisms.

For the purpose of clarity and a concise description, features are described herein as part of the same or separate embodiments, however, it will be appreciated that the disclosure includes embodiments having combinations of all or some of the features described.

### EXAMPLES

Example 1 Combining melting curve and fragment length data for unambiguous species identification.

To detect and identify different microorganisms, DNA regions that are broadly conserved throughout taxonomical groups may be used when they have specific characteristics. Important characteristics are the presence of conserved regions in the DNA, which may be used to amplify the DNA of many different species, with the use of broad-range primers, and the presence of variable regions flanked by conserved regions, which may be used to discriminate different species. There are different methods to analyze the variable regions, with differing suitability based on the specific characteristics of the variable region. A DNA region that is present in all life forms and harbors interspersed conserved and variable regions is the ribosomal DNA (rDNA). Within bacterial ribosomal DNA, the 16S-23S interspace (IS) region is of particular interest, as it is present in almost all bacteria, shows great variability in sequence composition and length and is flanked by highly conserved regions, which are highly suitable for targeting by broad-range primers.

Here we will demonstrate a very efficient and highly accurate method to detect and identify bacteria based on the combination of melting curve and fragment length analysis of the 16S-23S IS region.

Melting curves of the 16S-23S Interspace (IS) fragment in bacteria can be used to discriminate different bacterial species. Additionally, length measurements can also discriminate different bacterial species. While melting curve analysis or fragment analysis alone may be applied to identify bacterial species, there are many instances where this is not possible. In these instances, either fragment profiles are identical between species or melting curves do not give enough discrimination between species. When combining fragment length analysis with melting curve analysis, and comparing results to a database, unambiguous species identification can be made. Here, we will demonstrate that the combination of fragment length analysis with melting curve analysis can unambiguously discriminate bacteria up to the species level, where either fragment length analysis or melting curve analysis alone are unable to do so.

### Materials and methods

### Strain selection and cultivation

Clinical isolates of twelve different species were used in this analysis: Bacteroides fragilis, Bacteroides thetaiotaomicron, Enterococcus faecalis, Escherichia coli, Staphylococcus epidermidis, Staphylococcus aureus, Streptococcus cristatus, Streptococcus pyogenes, Corynebacterium jeikeium, Pseudomonas aeruginosa, Klebsiella pneumoniae and Enterobacter cloaace. Aerobic bacteria were cultured at 37°C on sheep blood agar (BioMerieux), incubated for 2 days aerobically, anaerobic bacteria were cultured on Schaedler agar (Oxoid), incubated for 3 days anaerobically. Identification of bacterial colonies was done by MALDI-TOF (VITEK MS system, Biomérieux).

### DNA isolation

DNA was isolated by adding 200µl of suspended bacteria (0,1 McFarland) to 400µl AL buffer (Qiagen, Hilden, Germany) and 40µl proteinase K in an Eppendorf container. This mixture was centrifuged for 10 seconds at 9000 g, then vortexed and incubated at 56°C while shaking at 1400 rpm for 1h. An easyMAG automated DNA isolation machine (Biomérieux) was used for further DNA extraction.

Sample mixtures (640 pl) were transported to an 8-welled easyMAG container and suspended in 2 ml nucliSENS lysis buffer as provided by the manufacturer. After incubation at room temperature for 1h, 70 pl of magnetic silica beads were added, as provided with the easyMAG machine. Afterward, the mixture was inserted in the easyMAG machine, and the "specific A" protocol was chosen, selecting the off-board workflow and eluting DNA in 110µl of NucliSens easyMAG extraction buffer 3 as provided by the manufacturer (Biomérieux). All DNA was stored at 4°C.

### PCR

Three different PCRs were used for the various analyses: the first two PCRs were used for phylum-specific amplification of 16S-23S regions and fragment analysis, the third PCR was used for universal amplification of 16S-23S regions, fragment analysis and melting curve analysis. The first PCR contained two different fluorescently labeled forward primers (SEQ ID NO: 1 and 2) targeting different bacterial groups and three reverse primers providing universal coverage for those groups (SEQ ID NOs:3-5). The first forward primer (SEQ ID NO:1) was specific for the phyla Firmicutes, Actinobacteria, Fusobacteria and Verrucomicrobia (FAFV) and the second labeled forward primer (SEQ ID NO:2) was specific for the phylum Bacteroidetes. The second PCR with a labeled forward primer (SEQ ID NO:6) combined with seven reverse primers (SEQ ID NOs:7-13) was specific for the phylum Proteobacteria (See Table 1). The third PCR reaction mix contained a set of primers recognizing a broad collection of bacteria: FOR: 5' CGGTGAATACGTTCCCGGIIIIIGTACAC 3' (SEQ ID NO: 14) and REV 5' CGTCCTTCDTCGVCTBIIIIIGCCARG-3' (SEQ ID NO: 15). The inosines bind to all 4 DNA bases, ensuring broad reactivity. The FOR primer contains an ATTO550 fluorescent moiety. The PCR reaction mix contains EvaGreen, which is an intercalating dye, that becomes fluorescent upon binding to double-stranded DNA.

Amplifications of the first two PCRs were carried out on a GeneAmp PCR system9700 (Applied Biosystems, Foster City, CA).

### Melting curve analysis

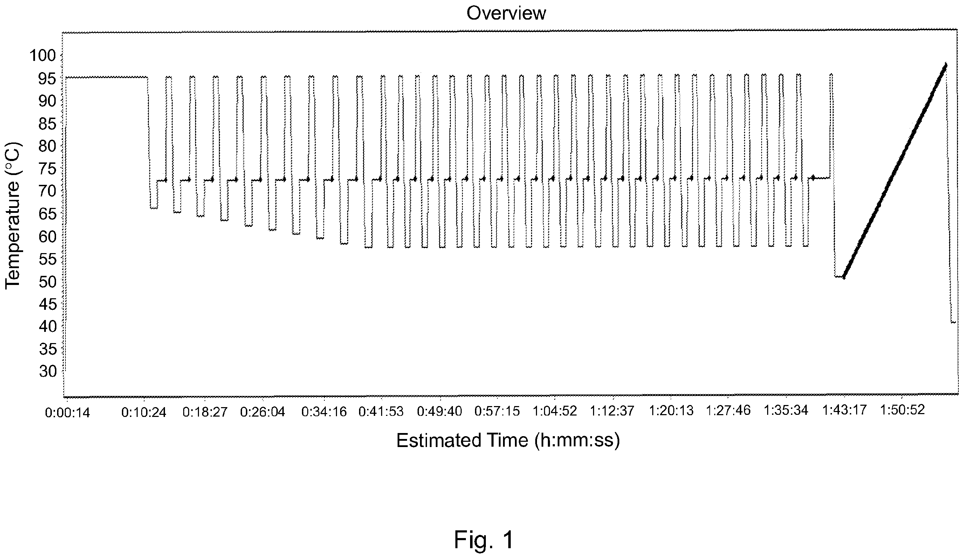

The third PCR included the melting curve analysis and was performed in a LightCycler480 (Roche) using the cycling schedule shown in Figure 1 (cycling schedule was identical for all three PCRs, only a melting curve analysis was added on the lightCycler machine).

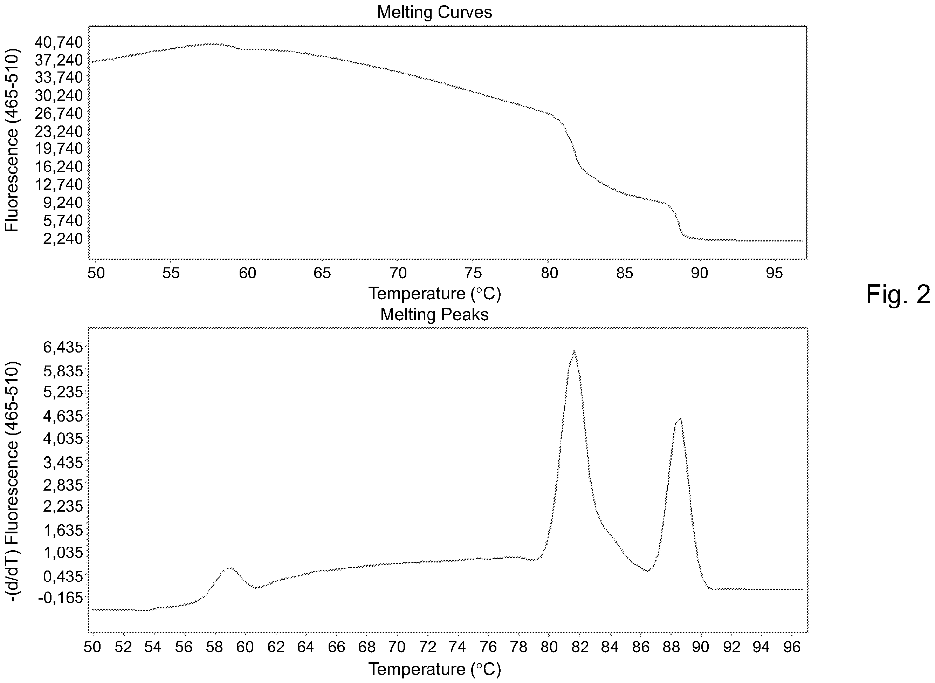

The program ends with a melting curve, where all PCR amplicons are slowly heated. The temperature at which the DNA strands part is dependent on sequence and length. A derivative of the fluorescence is then converted to 1 of more melting temperatures of the PCR product. For an example, see figure 2.

Species identification by melting curve analysis can be done by comparison to a proprietary melting curve database (inBiome).

### Fragment length analysis

After PCR, 5pl of PCR product was mixed with 20pl eMix (inBiome). DNA fragment analysis was performed on an ABI Prism 3500 Genetic Analyzer (Applied Biosystems). Data were analyzed with the inBiome proprietary software suite (inBiome, Amsterdam, the Netherlands) and results presented as microbial profiles. Automated species calling of fragment length profiles was done with a dedicated software suite (inBiome) in which peaks are linked to a database containing IS-profile information of >500 microbial species.

Peaks lower than 128RFU were regarded as background noise and were discarded from further analysis. The whole procedure, from DNA isolation to analyzed data could be done within four hours.

### Results

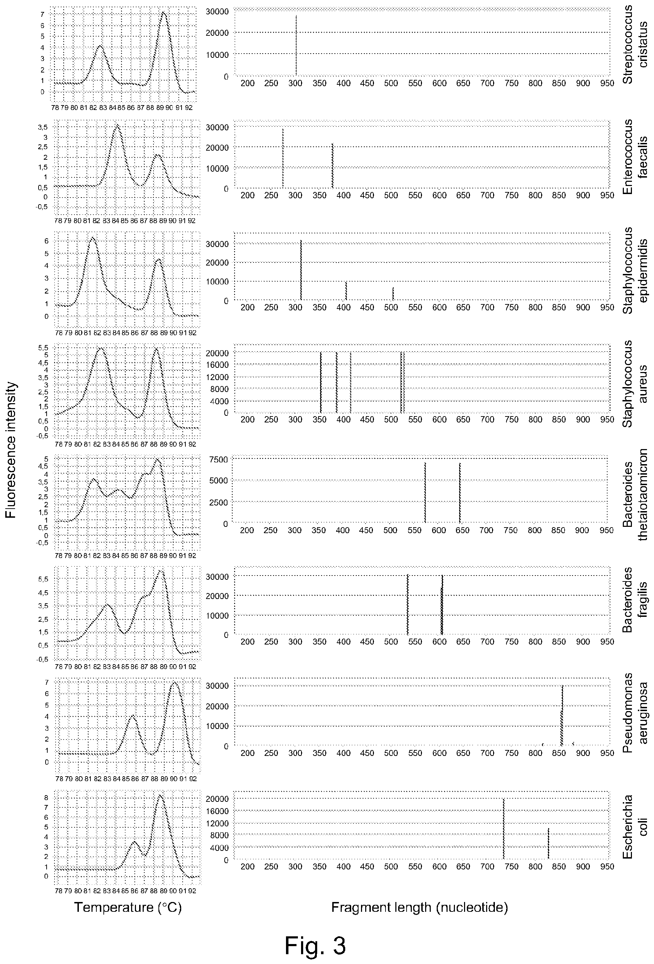

Melting curves and fragment analyses of the 16S-23S IS region of eight different species from three different bacterial phyla was performed in order to demonstrate the differential potential of the combination of melting curve and fragment length data. Results are shown in figure 3. Here, it can be clearly seen that melting curves and fragment length profiles are highly variable between species, even between different species within the same genus. By comparing these combined melting curve and fragment length data to a database containing reference profiles of known species (inBiome), an unambiguous identification could be made to the species level for each species in this analysis.

### Example 2 Accurate identification in the case of identical fragment lengths.

The situation may occur that when either a melting curve or a fragment length profile of a species is similar to that of another species. By combining melting curve data with fragment length data an unambiguous identification can still be made. An exemplary, real-life, situation in which fragment length profiles are similar is given in this example.

While the length of DNA fragments from the 16S-23S Interspace region are highly specific for each individual species, there are cases where different species have an identical fragment length signature. In these cases, while fragment lengths may be identical, the nucleotide sequence within these fragments are necessarily different: modern taxonomy is largely based on the sequence of ribosomal DNA and different species always have different nucleotide sequences in this region, especially in the interspace (IS) regions, which are the most variable regions.

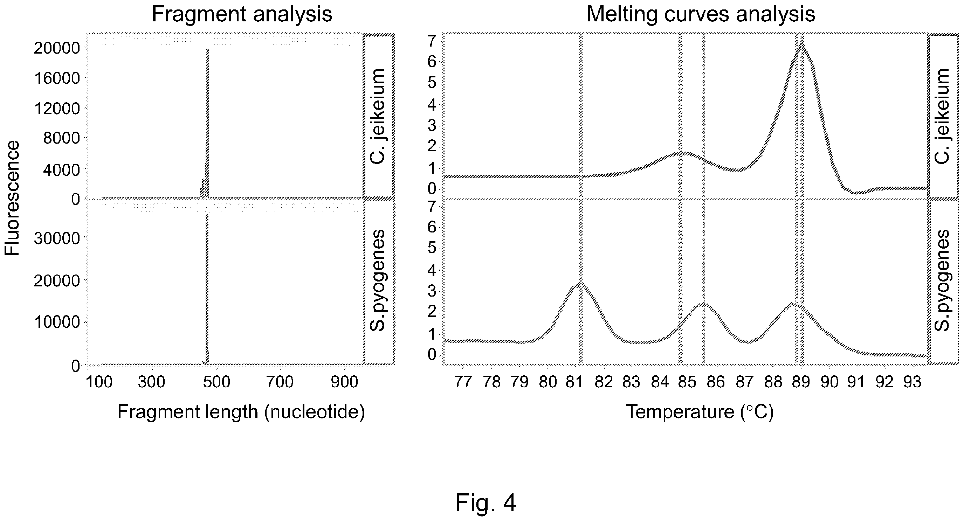

Melting temperature of a fragment is determined by two factors: length and sequence of a fragment. Therefore, when fragment lengths of two different species is identical, the sequence is necessarily different and therefore the melting curve signature has to be different. This situation is displayed in Figure 4, for two clinically highly relevant bacterial species, *Streptococcus pyogenes* and *Corynebacterium jeikeium*, for which species-level identification is crucial for correct treatment of patients.

It is shown that the two species can be accurately distinguished and identified using the methods of this invention.

### Example 3 Accurate identification in the case of identical melting curves.

Since melting curves are dependent on length and sequence of fragments, it is possible that, while lengths and sequences differ, the interplay between length and sequence might still give rise to similar melting curves.

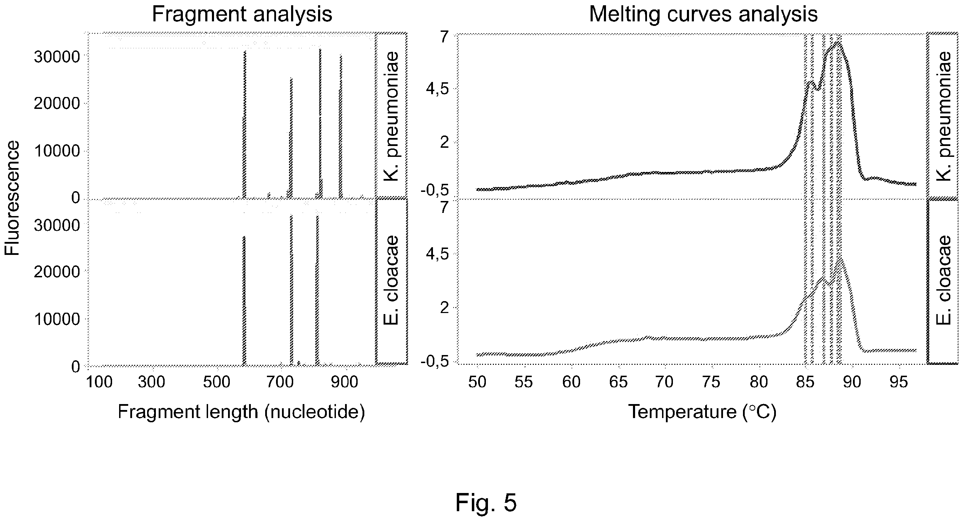

Similarity in melting curves may additionally be caused by the presence of multiple (PCR) fragments, of which the individual melting curves are superimposed on one another. This gives rise to complex melting curves, which may show a reduced resolution. This phenomenon can be particularly problematic in closely related species in which each species harbors multiple rRNA operons, and thus 16S-23S IS PCR fragments. In these cases, combining melting curve data with fragment length analysis can still uniquely identify each bacterial species. An exemplary, real-life, situation of this is illustrated in the analysis of the 16S-23S IS profiles of closely related species in the family of Enterobacteriaceae, a highly relevant bacterial family in clinical microbiology, containing many human pathogens. In the figure below, melting curves and fragment length analyses of *Enterobacter cloacae* and *Klebsiella pneumoniae* are shown. While melting curves are difficult to discriminate because of many similar IS-fragments in these closely related species, a unique length profile can still discriminate these species (See Figure 5).

**Table 1 SEQUENCES**

| **Primers for phyla Firmicutes and Bacteroidetes** | | | | | |
|---|---|---|---|---|---|
| **Primers** | **Type** | | **Sequence** | | **SEQ ID NO** |
| FirlSf | Forward | 5' - | CTGGATCACCTCCTTTCTAWG | - 3' | 1 |
| BacISf | Forward | 5' - | CTGGAACACCTCCTTTCTGGA | - 3' | 2 |
| DUISr1 | Reverse | 5' - | AGGCATCCACCGTGCGCCCT | - 3' | 3 |
| DUISr2 | Reverse | 5' - | AGGCATTCACCRTGCGCCCT | - 3' | 4 |
| DUISr3 | Reverse | 5' - | AGGCATCCRCCATGCGCCCT | - 3' | 5 |
| | | | | | |

| **Primers for phylum Proteabacteria** | | | | | |
|---|---|---|---|---|---|
| **Primer** | **Type** | | **Sequence** | | **SEQ ID NO** |
| ProtISf | Forward | 5' - | CCGCCCGTCACACCATGG | - 3' | 6 |
| DPISr1 | Reverse | 5` - | AATCTCGGTTGATTTGTTTTCCT | - 3' | 7 |
| DPISr2 | Reverse | 5' - | AATCTCGGTTGATTTCTTCTCCT | - 3' | 8 |
| DPISr3 | Reverse | 5' - | AATCTCTTTTGATTTCTTTTCCTCG | - 3' | 9 |
| DPISr4 | Reverse | 5' - | AATCTCATTTGATGTCTTTTCCTCG | - 3' | 10 |
| DPISr5 | Reverse | 5' - | AATCTCTTTTGATTTCTTTTCCTTCG | - 3' | 11 |
| DPISr6 | Reverse | 5' - | AATCTCTCTTGATTTCTTTTCCTTCG | - 3' | 12 |
| DPISr7 | Reverse | 5' - | AATCTCAATTGATTTCTTTTCCTAAGG | - 3' | 13 |
| | | | | | |

| **Universal bacterial primers (UNIMEC)** | | | | | |
|---|---|---|---|---|---|
| **Primer** | **Type** | | **Sequence** | | **SEQ ID NO** |
| ITS1FD | Forward | 5' - | CGGTGAATACGTTCCCGGIIIIIGTACAC | - 3' | 14 |
| ITS2RD | Reverse | 5' - | CGTCCTTCDTCGVCTBIIIIIGCCARG | - 3` | 15 |
| | | | | | |

| **Internal control primers** | | | | | |
|---|---|---|---|---|---|
| **Primer** | **Type** | | **Sequence** | | **SEQ ID NO** |
| **ICISf** | Forward | 5' - | GACCTAGTGGAGGAAAGATAC | - 3' | 16 |
| **ICISr** | Reverse | 5' - | GTAGGTGGCACGCGGGA | - 3' | 17 |

## Claims

1. A method for identifying a microorganism at species or strain level in a biological sample by polymerase chain reaction (PCR), said method comprising the steps of:
a) providing a biological sample that has been obtained and which is suspected of comprising microbes, and optionally isolating nucleic acid sequences comprised in said biological sample;
b) PCR amplifying at least one microbial rRNA internal transcribed spacer (ITS) region comprised in said, optionally isolated, nucleic acid sequences using a set of broad-taxonomic range amplification primers for amplifying said at least one microbial rRNA ITS, to thereby generate PCR amplicons;
c) recording a high resolution melting curve for the PCR amplicons generated in step b), and recording the length of the PCR amplicons generated in step b);
d) comparing the high resolution melting curve recorded in step c) with a database comprising high resolution melting curves of reference amplicons generated from reference microbial species or strains of known taxonomic identity using the same set of amplification primers, to thereby obtain a first taxonomic identity indicator of a microorganism present in said sample;
e) comparing the length of each PCR amplicon having a distinct length recorded in step c) with a database comprising PCR amplicon lengths of reference amplicons generated from reference microbial species or strains of known taxonomic identity using the same set of amplification primers, to thereby obtain a second taxonomic identity indicator of a microorganism present in said sample;
f) identifying a microorganism present in said sample to the species or strain level if the first and second taxonomic identity indicator match.

2. The method according to claim 1, wherein the biological sample suspected of comprising microbes is a biological sample suspected of comprising archaea, bacteria, viruses, protozoa, or fungi, or a combination thereof.

3. The method according to claim 1 or 2, wherein the reference amplicons in said database comprise amplicons generated by an *in vivo* and/or *in silico* PCR amplification reaction for amplifying the corresponding rRNA ITS region of a reference microbial species or strain of known taxonomic identity using the same set of broad-taxonomic range amplification primers for amplifying said at least one microbial rRNA ITS region.

4. The method according to any one of the preceding claims, wherein said databases in step d) and e) are combined into a single database, preferably wherein said database comprises rRNA ITS sequences and corresponding taxonomic identity data on bacteria.

5. The method according to any one of the preceding claims, wherein said broad-taxonomic range amplification primers are for amplifying a microbial rRNA ITS region of multiple, preferably essentially all, strains or species from a microbial genus, family, order, class, phylum, kingdom and/or domain, preferably essentially all strains or species from a microbial phylum, more preferably essentially all strains or species from a microbial kingdom, most preferably bacteria.

6. The method according to any one of the preceding claims, wherein said broad-taxonomic range amplification primers for amplifying said at least one microbial rRNA ITS region comprise a forward and reverse primer for amplifying a 16S-23S rRNA ITS region, a 23S-5S rRNA ITS region, a microbial 18S-5.8S rRNA ITS region, or a microbial 5.8S-26S/28S rRNA ITS region, preferably a forward and reverse primer for amplifying a 16S-23S rRNA ITS region.

7. The method according to any one of the preceding claims, wherein the biological sample is a bodily sample of a subject selected from a bodily fluid or exudate, including but not limited to uterine fluid, whole blood, serum, plasma, lymph fluid, mucus, saliva, sputum, stool/feces, sweat, wound fluid, pus/purulence, gastric content, ascites/ascitic fluid, bile, urine, semen, cerebrospinal fluid/liquor, and breast milk; or a bodily sample of a subject in the form of a swab, a biopsy, a lavage, or paper point sample, including but not limited to a sample from the skin, an organ, a tissue, the oral cavity, the urogenital tract, the vaginal tract, the gastrointestinal tract, respiratory tract or pulmonary system, and the cardiovascular system.

8. The method according to any one of the preceding claims, wherein the set of broad-taxonomic range amplification primers is a set of amplification primers for amplifying at least one rRNA ITS region of bacteria of the phylum *Bacteriodetes* and/or *Firmicutes.*

9. The method according to any one of the preceding claims, wherein said set of broad-taxonomic range amplification primers comprises each of the amplification primers of SEQ ID NOs: 1 and 3-5, or each of the amplification primers of SEQ ID NOs: 2-5, or each of the amplification primers of SEQ ID NOs: 1-5.

10. The method according to any one of the preceding claims, wherein said set of broad-taxonomic range amplification primers comprises each of the amplification primers of SEQ ID NOs: 6 and 7-13.

11. The method according to any one of the preceding claims, wherein said set of broad-taxonomic range amplification primers is a set of universal bacterial amplification primers, said set preferably comprising each of the amplification primers of SEQ ID NOs: 14-15.

12. The method according to any one of the preceding claims, wherein in step c) the length of the PCR amplicons is recorded by capillary electrophoresis or sequencing.

13. The method according to any one of the preceding claims, wherein step c), and optionally also step b), is performed in a miniaturized device, preferably a lab-on-a-chip (LOC) device.

14. The method according to any one of the preceding claims, wherein said database comprising high resolution melting curves and PCR amplicon lengths of reference amplicons generated from reference microbial species or strains of known taxonomic identity, further comprises high resolution melting curves and PCR amplicon lengths of reference amplicons generated from human sequences as controls for aspecific amplicon generation using said set of broad-taxonomic range amplification primers.

15. The method according to any one of the previous claims, wherein set of broad-taxonomic range amplification primers for amplifying at least one microbial rRNA ITS region comprises a labelled forward and/or labelled reverse primer, preferably a labelled forward primer, more preferably a fluorescently labelled forward primer.

## Patentansprüche

1. Verfahren zum Identifizieren eines Mikroorganismus auf Spezies- oder Stammebene in einer biologischen Probe durch Polymerasekettenreaktion (PCR), wobei das Verfahren die folgenden Schritte umfasst:
a) Bereitstellen einer biologischen Probe, die erhalten wurde und von der vermutet wird, dass sie Mikroben umfasst, und, optional, Isolieren von Nukleinsäuresequenzen, die in der biologischen Probe umfasst sind;
b) PCR-Amplifizieren von mindestens einer Interner-transkribierter-Spacer-, ITS-, -Region von mikrobieller rRNA, die in den, optional isolierten, Nukleinsäuresequenzen umfasst ist, unter Verwendung eines Satzes von Amplifikationsprimern mit breitem taxonomischem Bereich zum Amplifizieren der mindestens einen ITS-Region der mikrobiellen rRNA, um dadurch PCR-Amplikone zu erzeugen;
c) Aufzeichnen einer hochauflösenden Schmelzkurve für die in Schritt b) erzeugten PCR-Amplikone und Aufzeichnen der Länge der in Schritt b) erzeugten PCR-Amplikone;
d) Vergleichen der in Schritt c) aufgezeichneten hochauflösenden Schmelzkurve mit einer Datenbank, die hochauflösende Schmelzkurven von Referenzamplikonen umfasst, die aus mikrobiellen Referenzspezies oder - stämmen mit bekannter taxonomischer Identität unter Verwendung desselben Satzes von Amplifikationsprimern erzeugt wurden, um dadurch einen ersten taxonomischen Identitätsindikator eines in der Probe vorhandenen Mikroorganismus zu erhalten;
e) Vergleichen der Länge jedes in Schritt c) aufgezeichneten PCR-Amplikons mit einer bestimmten Länge mit einer Datenbank, die PCR-Amplikonlängen von Referenzamplikonen umfasst, die aus mikrobiellen Referenzspezies oder -stämmen mit bekannter taxonomischer Identität unter Verwendung desselben Satzes von Amplifikationsprimern erzeugt wurden, um dadurch einen zweiten taxonomischen Identitätsindikator eines in der Probe vorhandenen Mikroorganismus zu erhalten;
f) Identifizieren eines in der Probe vorhandenen Mikroorganismus auf Spezies- oder Stammebene, wenn der erste und der zweite taxonomische Identitätsindikator übereinstimmen.

2. Verfahren nach Anspruch 1, wobei die biologische Probe, von der vermutet wird, dass sie Mikroben umfasst, eine biologische Probe ist, von der vermutet wird, dass sie Archaeen, Bakterien, Viren, Protozoen oder Pilze oder eine Kombination davon umfasst.

3. Verfahren nach Anspruch 1 oder 2, wobei die Referenzamplikone in der Datenbank Amplikone umfassen, die durch eine *in vivo* und/oder *in silico* erfolgte PCR-Amplifikationsreaktion zum Amplifizieren der entsprechenden ITS-Region der rRNA einer mikrobiellen Referenzspezies oder eines Stamms mit bekannter taxonomischer Identität unter Verwendung desselben Satzes von Amplifikationsprimern mit breitem taxonomischem Bereich zum Amplifizieren der mindestens einen ITS-Region der mikrobiellen rRNA erzeugt wurden.

4. Verfahren nach einem der vorhergehenden Ansprüche, wobei die Datenbanken in Schritt d) und e) zu einer einzigen Datenbank kombiniert sind, wobei die Datenbank vorzugsweise ITS-Sequenzen der rRNA und entsprechende taxonomische Identitätsdaten über Bakterien umfasst.

5. Verfahren nach einem der vorhergehenden Ansprüche, wobei die Amplifikationsprimer mit breitem taxonomischem Bereich zum Amplifizieren einer ITS-Region der mikrobiellen rRNA von mehreren, vorzugsweise im Wesentlichen allen, Stämmen oder Spezies aus einer mikrobiellen Gattung, Familie, Ordnung, Klasse und/oder Domäne und/oder einem mikrobiellen Phylum und/oder Reich, vorzugsweise von im Wesentlichen allen Stämmen oder Spezies aus einem mikrobiellen Phylum, besonders vorzugsweise von im Wesentlichen allen Stämmen oder Spezies aus einem mikrobiellen Reich, ganz besonders vorzugsweise Bakterien, dienen.

6. Verfahren nach einem der vorhergehenden Ansprüche, wobei die Amplifikationsprimer mit breitem taxonomischem Bereich zum Amplifizieren der mindestens einen ITS-Region der mikrobiellen rRNA einen Vorwärts- und Rückwärtsprimer zum Amplifizieren einer ITS-Region der 16S-23S-rRNA, einer ITS-Region der 23S-5S-rRNA, einer ITS-Region der mikrobiellen 18S-5.8S-rRNA oder einer ITS-Region der mikrobiellen 5.8S-26S/28S-rRNA umfassen, vorzugsweise einen Vorwärts- und Rückwärtsprimer zum Amplifizieren einer ITS-Region der 16S-23S-rRNA.

7. Verfahren nach einem der vorhergehenden Ansprüche, wobei die biologische Probe eine Körperprobe eines Subjekts ist, die ausgewählt ist aus einer Körperflüssigkeit oder einem Exsudat, insbesondere aus uteriner Flüssigkeit, Vollblut, Serum, Plasma, Lymphflüssigkeit, Schleim, Speichel, Sputum, Stuhl/Fäkalien, Schweiß, Wundflüssigkeit, Eiter/Purulenz, Mageninhalt, Aszites/Aszitesflüssigkeit, Galle, Urin, Samen, Hirn-Rückenmarks-Flüssigkeit/Liquor und Muttermilch; oder eine Körperprobe eines Subjekts in Form eines Abstrichs, einer Biopsie, einer Lavage oder einer Papierspitzenprobe, insbesondere einer Probe von der Haut, einem Organ, einem Gewebe, der Mundhöhle, dem Urogenitaltrakt, dem Vaginaltrakt, dem Magen-Darm-Trakt, dem Respirationstrakt oder dem Lungensystem und dem Herz-Kreislauf-System.

8. Verfahren nach einem der vorhergehenden Ansprüche, wobei der Satz von Amplifikationsprimern mit breitem taxonomischem Bereich ein Satz von Amplifikationsprimern zum Amplifizieren von mindestens einer ITS-Region der rRNA von Bakterien des Phylums *Bacteriodetes* und/oder *Firmicutes* ist.

9. Verfahren nach einem der vorhergehenden Ansprüche, wobei der Satz von Amplifikationsprimern mit breitem taxonomischem Bereich jeden der Amplifikationsprimer mit den SEQ ID NO:1 und 3-5 oder jeden der Amplifikationsprimer mit den SEQ ID NO:2-5 oder jeden der Amplifikationsprimer mit den SEQ ID NO:1-5 umfasst.

10. Verfahren nach einem der vorhergehenden Ansprüche, wobei der Satz von Amplifikationsprimern mit breitem taxonomischem Bereich jeden der Amplifikationsprimer mit den SEQ ID NO:6 und 7-13 umfasst.

11. Verfahren nach einem der vorhergehenden Ansprüche, wobei der Satz von Amplifikationsprimern mit breitem taxonomischem Bereich ein Satz von universellen bakteriellen Amplifikationsprimern ist, wobei der Satz vorzugsweise jeden der Amplifikationsprimer mit den SEQ ID NO:14-15 umfasst.

12. Verfahren nach einem der vorhergehenden Ansprüche, wobei in Schritt c) die Länge der PCR-Amplikone durch Kapillarelektrophorese oder Sequenzierung aufgezeichnet wird.

13. Verfahren nach einem der vorhergehenden Ansprüche, wobei Schritt c) und optional auch Schritt b) in einer miniaturisierten Vorrichtung, vorzugsweise einer Lab-on-a-Chip-, LOC-, -Vorrichtung, durchgeführt wird.

14. Verfahren nach einem der vorhergehenden Ansprüche, wobei die Datenbank, die hochauflösende Schmelzkurven und PCR-Amplikonlängen von Referenzamplikonen umfasst, die aus mikrobiellen Referenzspezies oder -stämmen mit bekannter taxonomischer Identität erzeugt wurden, ferner hochauflösende Schmelzkurven und PCR-Amplikonlängen von Referenzamplikonen umfasst, die aus menschlichen Sequenzen als Kontrollen für die aspezifische Amplikonerzeugung unter Verwendung des Satzes von Amplifikationsprimern mit breitem taxonomischem Bereich erzeugt wurden.

15. Verfahren nach einem der vorhergehenden Ansprüche, wobei der Satz von Amplifikationsprimern mit breitem taxonomischem Bereich zum Amplifizieren von mindestens einer ITS-Region der mikrobiellen rRNA einen markierten Vorwärts- und/oder markierten Rückwärtsprimer, vorzugsweise einen markierten Vorwärtsprimer, besonders vorzugsweise einen fluoreszenzmarkierten Vorwärtsprimer, umfasst.

## Revendications

1. Procédé d'identification d'un micro-organisme au niveau de l'espèce ou de la souche dans un échantillon biologique par réaction en chaîne par polymérase (PCR), ledit procédé comprenant les étapes consistant à :
a) fournir un échantillon biologique qui a été obtenu et qui est suspecté de comprendre des microbes, et facultativement isoler des séquences d'acide nucléique comprises dans ledit échantillon biologique ;
b) amplifier par PCR en au moins une région d'espaceur transcrit interne d'ARNr microbien (ITS) comprise dans lesdites séquences d'acide nucléique, facultativement isolées, à l'aide d'un ensemble d'amorces d'amplification à large plage taxonomique pour amplifier ledit au moins un ITS d'ARNr microbien, pour ainsi générer des amplicons de PCR ;
c) enregistrer une courbe de fusion à haute résolution pour les amplicons de PCR générés à l'étape b), et enregistrer la longueur des amplicons de PCR générés à l'étape b) ;
d) comparer la courbe de fusion à haute résolution enregistrée à l'étape c) avec une base de données comprenant des courbes de fusion à haute résolution d'amplicons de référence générés à partir d'espèces microbiennes ou de souches de référence d'identité taxonomique connue à l'aide du même ensemble d'amorces d'amplification, pour ainsi obtenir un premier indicateur d'identité taxonomique d'un micro-organisme présent dans ledit échantillon ;
e) comparer la longueur de chaque amplicon de PCR ayant une longueur distincte enregistrée à l'étape c) avec une base de données comprenant des longueurs d'amplicon de PCR d'amplicons de référence générés à partir d'espèces microbiennes ou de souches de référence d'identité taxonomique connue à l'aide du même ensemble d'amorces d'amplification, pour ainsi obtenir un second indicateur d'identité taxonomique d'un micro-organisme présent dans ledit échantillon ;
f) identifier un micro-organisme présent dans ledit échantillon au niveau de l'espèce ou de la souche si les premier et second indicateurs d'identité taxonomique correspondent.

2. Procédé selon la revendication 1, dans lequel l'échantillon biologique suspecté de comprendre des microbes est un échantillon biologique suspecté de comprendre des archées, des bactéries, des virus, des protozoaires ou des champignons, ou une combinaison de ceux-ci.

3. Procédé selon la revendication 1 ou 2, dans lequel les amplicons de référence dans ladite base de données comprennent des amplicons générés par une réaction d'amplification par PCR in vivo et/ou in silico pour amplifier la région d'ITS d'ARNr correspondante d'une espèce ou d'une souche microbienne de référence d'identité taxonomique connue à l'aide du même ensemble d'amorces d'amplification à large plage taxonomique pour amplifier ladite au moins une région d'ITS d'ARNr microbien.

4. Procédé selon l'une quelconque des revendications précédentes, dans lequel lesdites bases de données à l'étape d) et e) sont combinées en une base de données unique, de préférence dans lequel ladite base de données comprend des séquences d'ITS d'ARNr et des données d'identité taxonomique correspondantes sur des bactéries.

5. Procédé selon l'une quelconque des revendications précédentes, dans lequel lesdites amorces d'amplification à large plage taxonomique sont destinées à amplifier une région d'ITS d'ARNr microbien de multiples souches ou espèces, de préférence sensiblement toutes, d'un genre, d'une famille, d'un ordre, d'une classe, d'un phylum, d'un règne et/ou d'un domaine microbien, de préférence sensiblement toutes les souches ou espèces d'un phylum microbien, de manière plus préférée sensiblement toutes les souches ou espèces d'un règne microbien, de la manière la plus préférée des bactéries.

6. Procédé selon l'une quelconque des revendications précédentes, dans lequel lesdites amorces d'amplification à large plage taxonomique pour amplifier ladite au moins une région d'ITS d'ARNr microbien comprennent une amorce directe et inverse pour amplifier une région d'ITS d'ARNr 16S-23S, une région d'ITS d'ARNr 23S-5S, une région d'ITS d'ARNr microbien 18S-5.8S, ou une région d'ITS d'ARNr microbien 5.8S-26S/28S, de préférence une amorce directe et inverse pour amplifier une région d'ITS d'ARNr 16S-23S.

7. Procédé selon l'une quelconque des revendications précédentes, dans lequel l'échantillon biologique est un échantillon corporel d'un sujet choisi parmi un fluide ou un exsudat corporel, y compris, mais sans s'y limiter, le fluide utérin, le sang entier, le sérum, le plasma, le fluide lymphatique, le mucus, la salive, les expectorations, les selles/excréments, la sueur, le fluide de plaie, le pus/la purulence, le contenu gastrique, les ascites/le fluide ascitique, la bile, l'urine, le sperme, le fluide/la liqueur cérébrospinal(e) et le lait maternel ; ou un échantillon corporel d'un sujet sous la forme d'un écouvillon, d'une biopsie, d'un lavage ou d'un échantillon ponctuel de papier, y compris, mais sans s'y limiter, un échantillon de la peau, d'un organe, d'un tissu, de la cavité buccale, du tractus urogénital, du tractus vaginal, du tractus gastro-intestinal, du tractus respiratoire ou du système pulmonaire et du système cardiovasculaire.

8. Procédé selon l'une quelconque des revendications précédentes, dans lequel l'ensemble d'amorces d'amplification à large plage taxonomique est un ensemble d'amorces d'amplification pour amplifier au moins une région d'ITS d'ARNr de bactéries du phylum *Bacteriodetes* et/ou *Firmicutes.*

9. Procédé selon l'une quelconque des revendications précédentes, dans lequel ledit ensemble d'amorces d'amplification à large plage taxonomique comprend chacune des amorces d'amplification des SEQ ID NOs : 1 et 3 à 5, ou chacune des amorces d'amplification des SEQ ID NOs : 2 à 5, ou chacune des amorces d'amplification des SEQ ID NOs : 1 à 5.

10. Procédé selon l'une quelconque des revendications précédentes, dans lequel ledit ensemble d'amorces d'amplification à large plage taxonomique comprend chacune des amorces d'amplification de SEQ ID NOs : 6 et 7 à 13.

11. Procédé selon l'une quelconque des revendications précédentes, dans lequel ledit ensemble d'amorces d'amplification à large plage taxonomique est un ensemble d'amorces d'amplification bactérienne universelle, ledit ensemble comprenant de préférence chacune des amorces d'amplification des SEQ ID NOs : 14 et 15.

12. Procédé selon l'une quelconque des revendications précédentes, dans lequel à l'étape c), la longueur des amplicons de PCR est enregistrée par électrophorèse capillaire ou séquençage.

13. Procédé selon l'une quelconque des revendications précédentes, dans lequel l'étape c), et facultativement également l'étape b), est réalisée dans un dispositif miniaturisé, de préférence un dispositif de laboratoire sur puce (LOC).

14. Procédé selon l'une quelconque des revendications précédentes, dans lequel ladite base de données comprenant des courbes de fusion à haute résolution et des longueurs d'amplicon de PCR d'amplicons de référence générés à partir d'espèces ou de souches microbiennes de référence d'identité taxonomique connue comprend en outre des courbes de fusion à haute résolution et des longueurs d'amplicon de PCR d'amplicons de référence générés à partir de séquences humaines en tant que témoins pour une génération d'amplicon aspécifique à l'aide dudit ensemble d'amorces d'amplification à large plage taxonomique.

15. Procédé selon l'une quelconque des revendications précédentes, dans lequel l'ensemble d'amorces d'amplification à large plage taxonomique pour amplifier au moins une région d'ITS d'ARNr microbien comprend une amorce avant marquée et/ou une amorce inverse marquée, de préférence une amorce avant marquée, de manière plus préférée une amorce avant marquée par fluorescence.
